(19) Europäisches Patentamt
European Patent Office
Office européen des brevets

(11) **EP 4 775 210 A1**

(12) **EUROPEAN PATENT APPLICATION**
published in accordance with Art. 153(4) EPC

(43) Date of publication:
**15.07.2026 Bulletin 2026/29**

(21) Application number: **23951271.8**

(22) Date of filing: **08.10.2023**

(51) International Patent Classification (IPC):
***A61K 31/7048*** *(2006.01)* ***A61K 31/122*** *(2006.01)*
***A61P 11/00*** *(2006.01)*

(52) Cooperative Patent Classification (CPC):
**A61K 31/12; A61K 31/7048; A61P 11/00**

(86) International application number:
**PCT/CN2023/123369**

(87) International publication number:
**WO 2025/050454 (13.03.2025 Gazette 2025/11)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.09.2023 CN 202311142999**

(71) Applicant: **Henan University of Chinese Medicine**
**Zhengzhou, Henan 450046 (CN)**

(72) Inventors:
- **LI, Jiansheng**
  **Zhengzhou, Henan 450046 (CN)**
- **TIAN, Yange**
  **Zhengzhou, Henan 450046 (CN)**
- **FENG, Suxiang**
  **Zhengzhou, Henan 450046 (CN)**
- **ZHAO, Peng**
  **Zhengzhou, Henan 450046 (CN)**
- **REN, Zhouxin**
  **Zhengzhou, Henan 450046 (CN)**
- **YU, Xueqing**
  **Zhengzhou, Henan 450046 (CN)**
- **LIU, Xuefang**
  **Zhengzhou, Henan 450046 (CN)**
- **DONG, Haoran**
  **Zhengzhou, Henan 450046 (CN)**
- **YANG, Shuguang**
  **Zhengzhou, Henan 450046 (CN)**

(74) Representative: **dompatent**
**Partnerschaft von**
**Patentanwälten und Rechtsanwälten mbB**
**Deichmannhaus am Dom**
**Bahnhofsvorplatz 1**
**50667 Köln (DE)**

# (54) COMPOSITION OF PAEONIFLORIN AND ISOLIQUIRITIGENIN AND USE THEREOF

(57) The present invention relates to a composition of paeoniflorin and isoliquiritigenin and application thereof. The composition according to the present invention is rich in raw materials, and has the effects of anti-inflammation, antioxidant, inhibiting collagen deposition, etc., and can be effectively used to treat diffuse interstitial pulmonary fibrosis with high economic and social benefits.

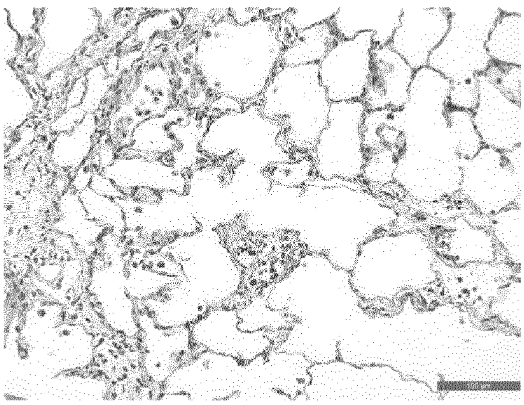

Fig. 74

EP 4 775 210 A1

## Description

### Technical field

The invention belongs to the technical field of drugs for treating diffuse interstitial pulmonary fibrosis, and relates to a composition of paeoniflorin and isoliquiritigenin and application thereof.

### Background

Diffuse interstitial pulmonary fibrosis, which is a group of diffuse lung diseases that involve the pulmonary interstitium and alveolar space and lead to the loss of alveolar-capillary functional units, has high disability and mortality rates, causing heavy social and economic burden and serious harm to public health. The disease is complex in causes and may be related to dust, harmful smoke, infection, drugs, etc., with the clinical manifestations of progressively aggravated dyspnea, restrictive ventilation disorder accompanied by reduced diffusing function, and eventually leading to respiratory failure. Among them, idiopathic pulmonary fibrosis (IPF) is the most representative.

IPF is the most common interstitial lung disease clinically. It is more common in men than in women, and more common in the elderly. Its prognosis is poor, and its incidence rate is increasing year by year. The prevalence rates of IPF in Italy, the United States, and the United Kingdom are 7.5~9.3/100,000, 14~42.7/100,000, and 10~25/100,000, respectively. The IPF patients after diagnosis have an average survival time of 2.5 to 3.5 years, and a significantly decreased survival rate over time with a 3-year survival rate of 50% and a 5-year survival rate of only 20%. IPF has main pathological features of diffuse alveolitis, alveolar structural disorder and interstitial thickening, with main pathological mechanisms of inflammatory response, oxidative stress, apoptosis, and epithelial-mesenchymal transition. Its pathological characteristics mainly includes a large number of fibroblast accumulation and extracellular matrix deposition accompanied by inflammation and injury, leading to structural changes in normal lung tissue and loss of function, mainly involving the pulmonary interstitium, alveoli, and bronchioles, which can ultimately lead to respiratory failure. Transforming growth factor (TGF)-β1 and interleukin (IL)-13 are important profibrotic factors that can promote the formation of fibrosis. Hydroxyproline (HYP) is the main component of collagen tissue, and its expression can reflect collagen deposition and degree of pulmonary fibrosis.

In 2015, the American Thoracic Society (ATS)/European Respiratory Society (ERS)/Japanese Respiratory Society (JRS)/Latin American Thoracic Society (ALAT) revised the 2011 IPF guidelines, pointing out that pirfenidone is a recommended drug. However, pirfenidone has a wide range of adverse reactions, and some patients still cannot tolerate certain adverse reactions even when the treatment is beneficial as assessed by forced vital capacity (FVC). In addition, pirfenidone treatment is expensive and difficult to promote clinically.

Therefore, it is necessary to further research and develop drugs for the treatment of diffuse interstitial pulmonary fibrosis to improve the level and ability of clinical prevention and treatment.

### Summary of the invention

The inventor team had developed a traditional Chinese medicine component formula consisting of ginsenoside Re, icariin, nobiletin, peimine, paeoniflorin and isoliquiritigenin for the treatment of diffuse interstitial pulmonary fibrosis, and filed an invention application for the same, which was granted a patent right (CN109394776B). Based on the above research, the inventors further found that a traditional Chinese medicine component composition consisting of paeoniflorin and isoliquiritigenin can achieve better therapeutic effects, thus completing the present invention.

In one aspect, the present invention provides a pharmaceutical composition consisting of paeoniflorin and isoliquiritigenin.

Paeoniflorin (CAS: 23180-57-6, molecular formula: $C_{23}H_{28}O_{11}$, molecular weight: 480.47 g/mol) has the following chemical structural formula:

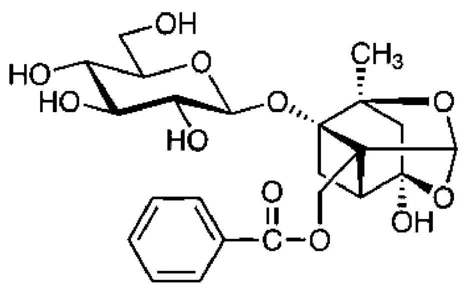

.

Isoliquiritigenin (CAS: 961-29-5, molecular formula: $C_{15}H_{12}O_4$, molecular weight: 256.25 g/mol) has the following chemical structural formula:

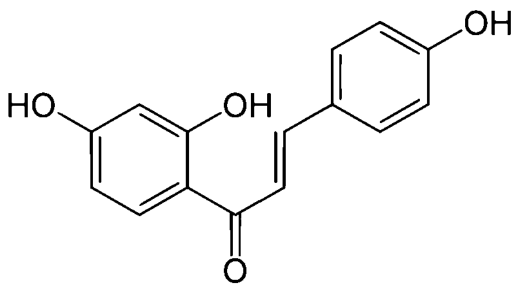

.

In the pharmaceutical composition according to the present invention, the ratio of paeoniflorin and isoliquiritigenin is not particularly limited, as long as a substantial synergistic effect can be achieved.

In some embodiments, in the pharmaceutical composition according to the present invention, the weight ratio of paeoniflorin and isoliquiritigenin can be 10:90 to 90: 10, such as 20:80 to 80:20, 30:70 to 70:30, 40:60 to 60:40, 40:60 to 55:45, or 40:60 to 50:50.

In some embodiments, in the pharmaceutical composition according to the present invention, based on the total weight of the pharmaceutical composition, the weight percentage of paeoniflorin can be 10% or more, 20% or more, 25% or more, 30% or more, 35% or more, or 40% or more, and 90% or less, 80% or less, 75% or less, 70% or less, 65% or less, 60% or less, 55% or less, or 50% or less, such as 40% to 50%.

In some embodiments, in the pharmaceutical composition according to the present invention, paeoniflorin and isoliquiritigenin have a purity of not less than 98%, as determined by high performance liquid chromatography. High-performance liquid chromatography is a conventional measurement method and is a well-known technology, so its description is omitted here.

As demonstrated in the Examples, the pharmaceutical composition according to the present invention can be used to prevent or treat diffuse interstitial pulmonary fibrosis, and can achieve better therapeutic effects compared with the traditional Chinese medicine component formula in CN109394776B.

In another aspect, the present invention provides the application of the above pharmaceutical composition for preparing a medicine for preventing or treating diffuse interstitial pulmonary fibrosis.

In yet another aspect, the present invention provides a medicine comprising the above pharmaceutical composition for preventing or treating diffuse interstitial pulmonary fibrosis.

In particular, the above-mentioned medicine does not comprise ginsenoside Re, icariin, nobiletin, and peimine.

Ginsenoside Re (CAS: 489-32-7, molecular formula: $C_{48}H_{82}O_{18}$, molecular weight: 947.154 g/mol) has the following chemical structural formula:

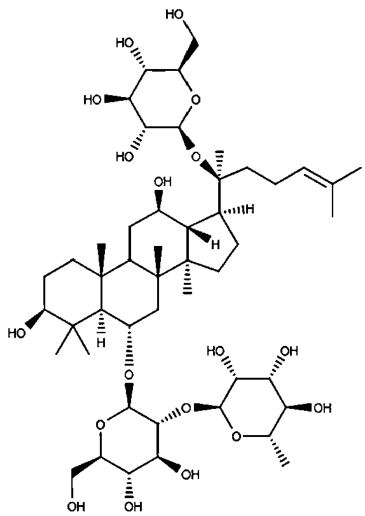

.

Icariin (CAS: 489-32-7, molecular formula: $C_{33}H_{40}O_{15}$, molecular weight: 676.65 g/mol) has the following chemical structural formula:

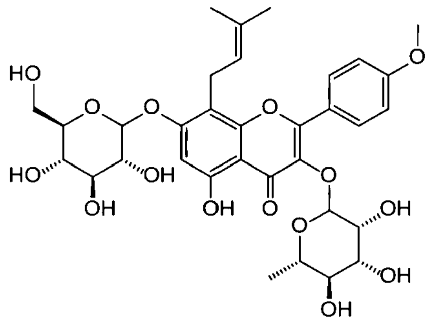

Nobiletin (CAS: 478-01-3, molecular formula: $C_{21}H_{22}O_8$, molecular weight: 402.39 g/mol) has the following chemical structural formula:

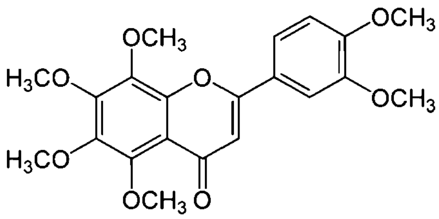

Peimine (CAS: 23496-41-5, molecular formula: $C_{27}H_{45}NO_3$, molecular weight: 431.65 g/mol) has the following chemical structural formula:

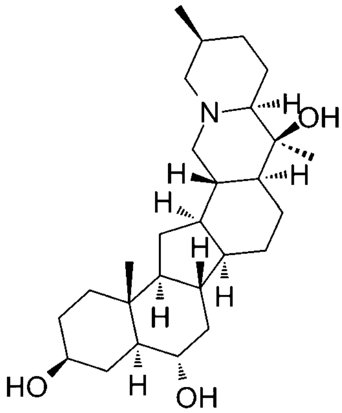

In further another aspect, the present invention provides a method for preventing or treating diffuse interstitial pulmonary fibrosis, comprising administering the pharmaceutical composition according to the present invention or the medicine according to the present invention to a subject in need thereof.

The subject here may be a human or an animal, such as a mammal, such as human, ape, monkey, pig, horse, cow, sheep, dog, cat, lion, tiger, etc., but is not limited thereto.

The medicine of the present invention can be in various conventional dosage forms in the art, such as in the form of a solid, semi-solid or liquid, for example, aqueous solutions, non-aqueous solutions, suspensions, lozenges, capsules, tablets, granules, pills, and powders and inhalants, etc. The medicine can be administered by injection, orally or inhalationaly. The injection may be intravenous, intramuscular, intraperitoneal, intradermal or subcutaneous injection, etc.

The medicine may comprise the pharmaceutical composition of the present invention in any amount, as long as the expected preventive or therapeutic benefit can be achieved. For example, the medicine may comprise 0.01-99.99%, such as 1% to 90% by mass of the pharmaceutical composition of the present invention, and 99.99-0.01%, such as 99% to 10% by mass of a pharmaceutically acceptable auxiliary.

The conventional pharmaceutically acceptable auxiliary may be selected from the group consisting of excipients, fillers, diluents, preservatives, colorants, flavoring agents, flavor agents, sustained release agents, coating agents, penetration enhancers, adhesives, wetting agents, disintegrants, surfactants, lubricants, solubilizers, fragrances, additives, buffers, soothing agents, etc., but are not limited thereto.

In addition, the medicine of the present invention may also comprise a further active ingredient according to the

needs of treatment or comorbidities and the like. The further active ingredient may be an active ingredient for the prevention or treatment of diffuse interstitial pulmonary fibrosis, or for the treatment of a further disease, disorder, or condition.

The dosage of the pharmaceutical composition and the medicine according to the present invention can be selected according to the administration guidelines, the age, gender and other conditions of the patient, and the severity of the disease.

The present invention has been described in details above, but the above-mentioned embodiments are only illustrative in nature and are not intended to limit the present invention. Furthermore, there is no intention to be bound by any theory described in the preceding Background or Summary of the invention, or in the following examples. Unless expressly stated otherwise, numerical ranges throughout this application include any subrange therein and any numerical value incrementing the smallest subunit of the value given therein. Unless expressly stated otherwise, numerical values throughout this application represent approximate measures or limitations of the scope of embodiments that include minor deviations from the given values and have approximately the recited value and the precise value recited. With the exception of the working examples provided at the end of the detailed description, all numerical values for parameters (eg quantities or conditions) in this application document (including the appended claims) are to be understood in all cases as being modified by the term " approximately ", regardless of whether "approximately " actually appears before the value. "Approximately" means that the stated value is allowed to be slightly less precise (somewhere near exact at the value; about or reasonably close to the value; approximation). If the imprecision provided by "approximately" is not understood in this ordinary sense in the art, "approximately" as used herein at least means the variation that can be produced by ordinary methods of measuring and using these parameters. For example, "approximately" may include a variation of less than or equal to 10%, less than or equal to 5%, less than or equal to 4%, less than or equal to 3%, less than or equal to 2%, less than or equal to 1%, or less than or equal to 0.5%.

## Beneficial effects

During the development, the inventors obtained same or similar results in repeated experiments, indicating that the method is effective and feasible. In addition, the present invention is rich in raw materials, and thus has good practical application value.

The composition for treating diffuse interstitial pulmonary fibrosis of the present invention is extracted and screened from Paeonia lactiflora and licorice. It has the effects of anti-inflammation, antioxidant, inhibiting collagen deposition, etc., and can be effectively used to treat diffuse interstitial pulmonary fibrosis. The present invention repeatedly studied the efficacy of the compositions in respective examples in treating diffuse pulmonary interstitial fibrosis in rats, and satisfactory therapeutic effects were achieved.

The composition of the present invention is rich in raw materials, is completed by a scientific and advanced method, and is effective in treating diffuse pulmonary interstitial fibrosis, thus it can be developed into a new drug for treating diffuse pulmonary interstitial fibrosis, with high economic and social benefits.

## Brief Description of the drawings

Fig. 1: H&E staining image of lung tissue of the rat in the model group in Example 1;
Fig. 2: H&E staining image of lung tissue of the rat in the blank group in Example 1;
Fig. 3: H&E staining image of lung tissue of the rat in the pirfenidone group in Example 1;
Fig. 4: H&E staining image of lung tissue of the rat in the group of Formula 1 in Example 1;
Fig. 5: H&E staining image of lung tissue of the rat in the group of Formula 2 in Example 1;
Fig. 6: H&E staining image of lung tissue of the rat in the group of Formula 3 in Example 1;
Fig. 7: H&E staining image of lung tissue of the rat in the group of Formula 4 in Example 1;
Fig. 8: H&E staining image of lung tissue of the rat in the group of Formula 5 in Example 1;
Fig. 9: H&E staining image of lung tissue of the rat in the group of Formula 6 in Example 1;
Fig. 10: H&E staining image of lung tissue of the rat in the group of Formula 7 in Example 1;
Fig. 11: H&E staining image of lung tissue of the rat in the group of Formula 8 in Example 1;
Fig. 12: H&E staining image of lung tissue of the rat in the group of Formula 9 in Example 1;
Fig. 13: H&E staining image of lung tissue of the rat in the group of Formula 10 in Example 1;
Fig. 14: H&E staining image of lung tissue of the rat in the group of Formula 11 in Example 1;
Fig. 15: H&E staining image of lung tissue of the rat in the group of Formula 12 in Example 1;
Fig. 16: H&E staining image of lung tissue of the rat in the group of Formula 13 in Example 1;
Fig. 17: H&E staining image of lung tissue of the rat in the group of Formula 14 in Example 1;

Fig. 18: Masson staining image of lung tissue of the rat in the model group in Example 1;
Fig. 19: Masson staining image of lung tissue of the rat in the blank group in Example 1;
Fig. 20: Masson staining image of lung tissue of the rat in the pirfenidone group in Example 1;
Fig. 21: Masson staining image of lung tissue of the rat in the group of Formula 1 in Example 1;
Fig. 22: Masson staining image of lung tissue of the rat in the group of Formula 2 in Example 1;
Fig. 23: Masson staining image of lung tissue of the rat in the group of Formula 3 in Example 1;
Fig. 24: Masson staining image of lung tissue of the rat in the group of Formula 4 in Example 1;
Fig. 25: Masson staining image of lung tissue of the rat in the group of Formula 5 in Example 1;
Fig. 26: Masson staining image of lung tissue of the rat in the group of Formula 6 in Example 1;
Fig. 27: Masson staining image of lung tissue of the rat in the group of Formula 7 in Example 1;
Fig. 28: Masson staining image of lung tissue of the rat in the group of Formula 8 in Example 1;
Fig. 29: Masson staining image of lung tissue of the rat in the group of Formula 9 in Example 1;
Fig. 30: Masson staining image of lung tissue of the rat in the group of Formula 10 in Example 1;
Fig. 31: Masson staining image of lung tissue of the rat in the group of Formula 11 in Example 1;
Fig. 32: Masson staining image of lung tissue of the rat in the group of Formula 12 in Example 1;
Fig. 33: Masson staining image of lung tissue of the rat in the group of Formula 13 in Example 1;
Fig. 34: Masson staining image of lung tissue of the rat in the group of Formula 14 in Example 1;
Fig. 35: CoL I staining image of lung tissue of the rat in the model group in Example 1;
Fig. 36: CoL I staining image of lung tissue of the rat in the blank group in Example 1;
Fig. 37: CoL I staining image of lung tissue of the rat in the pirfenidone group in Example 1;
Fig. 38: CoL I staining image of lung tissue of the rat in the group of Formula 1 in Example 1;
Fig. 39: CoL I staining image of lung tissue of the rat in the group of Formula 2 in Example 1;
Fig. 40: CoL I staining image of lung tissue of the rat in the group of Formula 3 in Example 1;
Fig. 41: CoL I staining image of lung tissue of the rat in the group of Formula 4 in Example 1;
Fig. 42: CoL I staining image of lung tissue of the rat in the group of Formula 5 in Example 1;
Fig. 43: CoL I staining image of lung tissue of the rat in the group of Formula 6 in Example 1;
Fig. 44: CoL I staining image of lung tissue of the rat in the group of Formula 7 in Example 1;
Fig. 45: CoL I staining image of lung tissue of the rat in the group of Formula 8 in Example 1;
Fig. 46: CoL I staining image of lung tissue of the rat in the group of Formula 9 in Example 1;
Fig. 47: CoL I staining image of lung tissue of the rat in the group of Formula 10 in Example 1;
Fig. 48: CoL I staining image of lung tissue of the rat in the group of Formula 11 in Example 1;
Fig. 49: CoL I staining image of lung tissue of the rat in the group of Formula 12 in Example 1;
Fig. 50: CoL I staining image of lung tissue of the rat in the group of Formula 13 in Example 1;
Fig. 51: CoL I staining image of lung tissue of the rat in the group of Formula 14 in Example 1;
Fig. 52: α-SMA staining image of lung tissue of the rat in the model group in Example 1;
Fig. 53: α-SMA staining image of lung tissue of the rat in the blank group in Example 1;
Fig. 54: α-SMA staining image of lung tissue of the rat in the pirfenidone group in Example 1;
Fig. 55: α-SMA staining image of lung tissue of the rat in the group of Formula 1 in Example 1;
Fig. 56: α-SMA staining image of lung tissue of the rat in the group of Formula 2 in Example 1;
Fig. 57: α-SMA staining image of lung tissue of the rat in the group of Formula 3 in Example 1;
Fig. 58: α-SMA staining image of lung tissue of the rat in the group of Formula 4 in Example 1;
Fig. 59: α-SMA staining image of lung tissue of the rat in the group of Formula 5 in Example 1;
Fig. 60: α-SMA staining image of lung tissue of the rat in the group of Formula 6 in Example 1;
Fig. 61: α-SMA staining image of lung tissue of the rat in the group of Formula 7 in Example 1;
Fig. 62: α-SMA staining image of lung tissue of the rat in the group of Formula 8 in Example 1;
Fig. 63: α-SMA staining image of lung tissue of the rat in the group of Formula 9 in Example 1;
Fig. 64: α-SMA staining image of lung tissue of the rat in the group of Formula 10 in Example 1;
Fig. 65: α-SMA staining image of lung tissue of the rat in the group of Formula 11 in Example 1;
Fig. 66: α-SMA staining image of lung tissue of the rat in the group of Formula 12 in Example 1;
Fig. 67: α-SMA staining image of lung tissue of the rat in the group of Formula 13 in Example 1;
Fig. 68: α-SMA staining image of lung tissue of the rat in the group of Formula 14 in Example 1;
Fig. 69: H&E staining of lung tissue of the rat in the model group in Example 2;
Fig. 70: H&E staining image of lung tissue of the rat in the blank group in Example 2;
Fig. 71: H&E staining image of lung tissue of the rat in the pirfenidone group in Example 2;
Fig. 72: H&E staining image of lung tissue of the rat in the isoliquiritigenin group in Example 2;
Fig. 73: H&E staining image of lung tissue of the rat in the paeoniflorin group in Example 2;
Fig. 74: H&E staining image of lung tissue of the rat in the combination group in Example 2;
Fig. 75: Masson staining image of lung tissue of the rat in the model group in Example 2;

Fig. 76: Masson staining image of lung tissue of the rat in the blank group in Example 2;
Fig. 77: Masson staining image of lung tissue of the rat in the pirfenidone group in Example 2;
Fig. 78: Masson staining image of lung tissue of the rat in the isoliquiritigenin group in Example 2;
Fig. 79: Masson staining image of lung tissue of the rat in the paeoniflorin group in Example 2;
Fig. 80: Masson staining image of lung tissue of the rat in the combination group in Example 2.

**Detailed Embodiments**

The specific embodiments of the present invention will be further described in detail below with reference to examples. However, the following examples are provided only for illustrating the present invention, and the scope of the present invention is not limited thereto.

**Example 1 Screening the formulas of the pharmaceutical compositions**

**1 Experimental materials**

**1.1 Experimental animals**

204 SPF grade SD rats, half male and half female (age: 6-8 weeks, weight: 200 + 10g; animal license number: 37009200018733), were purchased from Jinan Pengyue Experimental Animal Breeding Co., Ltd. (SCXK [Lu] 20140007).

**1.2 Experimental drugs**

1.2.1 Bleomycin hydrochloride for injection (batch number: s1214-12, 15U/bottle, Hisun Pfizer Pharmaceuticals Co., LTD.), dissolved in normal saline to be a 30 mg/ml solution.

1.2.2 Pirfenidone capsules (Aisre, 100 mg $\times$ 54 capsules/bottle, Beijing Continent Pharmaceuticals Co., Ltd), as the positive control, prepared with distilled water into a 33.3 mg/ml solution.

1.2.3 Icariin, isoliquiritigenin, peimine, nobiletin, paeoniflorin and ginsenoside were purchased from Chengdu Mansite Biotechnology Co., Ltd. Each of the above ingredients has a purity of higher than 98%, as determined by high performance liquid chromatography. Before use, the ingredients were respectively dissolved in a 0.5% solution of sodium carboxymethyl cellulose (CMC-Na) to prepare a suspension.

**1.3 Experimental reagents**

1.3.1 Immunohistochemistry (IHC) related reagents

SABC ready-to-use rabbit IgG immunohistochemistry kit (SA1022), DAB chromogenic kit (AR1022), and Mayor’s hematoxylin (AR0005) were provided by Wuhan BOSTER Biological Technology Co., Ltd.

Primary antibody rabbit anti-rat Anti-Collagen I (COL I) Antibody (Cat. No. AF7001) and Anti-alpha-SMA ($\alpha$-SMA) Antibody (Cat. No. AF1032) were provided by Affinity Biotech Co., Ltd., US.

$\alpha$-SMA (14395-1-AP) polyclonal antibody was provided by Wuhan Sanying Biotechnology Co., Ltd..

1.3.2 Hematoxylin-eosin (H&E) staining and Masson’s Trichrome staining related reagents

Paraformaldehyde (P804536) was provided by Shanghai Macklin Biochemical Technology Co., Ltd. (Shanghai, China).

Paraffin (39601006) was provided by Leica Microsystems Trading Co., Ltd. (Illinois, USA).

Absolute ethanol (500 ml) and xylene (500 ml) were provided by Tianjin Zhiyuan Chemical Reagent Co., Ltd.

Hematoxylin staining solution (G1140), eosin staining solution (G1100), and neutral gum (G8590) were provided by Beijing solarbio science & technology Co., Ltd. Masson dye (G1006) was provided by Wuhan Servicebio Technology Co., Ltd.

**1.4 Experimental instruments**

The experimental instruments are shown in Table 1.

Table 1

| Instrument | Model | Manufacturer | |
|---|---|---|---|
| Touch screen IVC-II type independent ventilated isolation cage and accessories | Fengshi IVC | Fengshi Laboratory Animal Equipment Co., Ltd | China |
| Small animal whole body plethysmography detection system | Fine Pointe ™ Whole Body Plethysmography 4-Site System | DSI | US |
| Fine Pointe TM series PFT measurement system | Fine Pointe ™ series PFT | DSI | US |
| Biological safety cabinet | 1374 | Thermo fisher scientific (suzhou) instruments co. Ltd. | China |
| High pressure desktop sterilizer | HVE-50 | HIRAYAMA | China |
| Ultrapure water system | Milli-Q | Merk Millipore | Germany |
| Small desktop high speed refrigerated centrifuge | Sorvall Legend Micro 17R Centrifuge | Thermo Scientific | US |
| Microplate reader | MULTISKAN GO | Thermo Scientific | US |
| Automatic slicer | RM2145 | Leica | Germany |
| Image-Pro Plus 6.0 image analysis software | | Media Cybernetics | US |
| Electronic balance | PL2001-L | Mettler Toledo | China |
| Microscopes and Photomicrography Systems | DP70 | Olympus | Japan |
| Ultra low temperature freezer | ULT-1386-3 | REVCO | US |
| Electric Thermostatic Drying Oven | DUG-9240A | Shanghai Jinghong Experimental Equipment Co. Ltd | China |
| Ice machine | AF100 | Scotsman | Italy |

## 2 Research methods

### 2.1 Model preparation

The purchased rats were allowed to adapt to the environment for 7 days. They were housed in individual ventilated cages (IVC) under the following conditions: (20-25) °C, (50 ± 10)% relative humidity, 15 times/h air exchange, ammonia concentration ≤ 14 $mg/m^3$, noise ≤ 60 db, and 12 h day and night cycle. The purification and water and electricity operating systems were regularly checked and the environment was kept quiet.

A rat pulmonary fibrosis model was prepared using a one-time intratracheal instillation of bleomycin. After the rat was anesthetized by intraperitoneal injection of 10% chloral hydrate (3.0 ml/kg), it was tilted back and fixed on a rat board and irradiated with light source on the throat, and the glottis was seen to open and close in an orderly manner with breathing when the tongue was gently lifted to open the mouth. The indwelling needle was gently pushed into the trachea as the glottis opened and closed, the needle core was pulled out, and whether tracheal intubation was successful was verified by swing of a cotton thread. Bleomycin was injected into the trachea at a dose of 5 mg/kg, and 0.5 ml of air was further injected to ensure the depth of the drug. The rat was held upright and rocked from side to side to ensure even distribution of the drug in the lung. The anesthetized rat was placed on an electric blanket to maintain body temperature. After waking up, it is placed into a corresponding cage. The rats were fed normally until the 28th day, drinking pure water and eating sterile feed freely every day.

### 2.2 Treatment

204 rats were divided randomly into blank group, model group, pirfenidone group (positive control) and respective formula groups (formulas 1-14), wherein the formula 1 is the traditional Chinese medicine component formula in CN109394776B, 17 groups in total, with 12 rats in each group, 6 female rats/group and 6 male rats/group. The modeling day was set as day 0. From the 29th day, the blank group (normal rats without modeling) and the model groups were intragastrically administered with physiological saline (2ml/rat), and the pirfenidone group was intragastrically administered with pirfenidone solution (108 $mg\ kg^{-1}d^{-1}$), and the respective formula groups were intragastrically administered (see

Table 3 for dose) once daily for 14 days, and the samples were collected at the end of $42^{nd}$ day.

The drug dose is calculated using the equivalent dose coefficient conversion formula below:

$$D_{rat} = D_{human} \times (HI_{rat}/HI_{human}) \times (W_{rat}/W_{human})^{2/3}$$

Wherein, D: dose, HI: body shape coefficient, W: body weight.

**2.3 Indicator detection**

2.3.1 General conditions:

The death, bleeding around the eyes, and bleeding of mouth and nose of the rats were observed. The rats' fur color, mental status, eating and activities, etc., were also observed every day.

2.3.2 Lung function:

Before collecting the sample, the rat was anesthetized and submitted to a tracheotomy, and the tracheal tube was inserted into the rat's trachea and fixed. The FinePointe™ series PFT measurement system was used to detect the rat's forced vital capacity (FVC), forced expiratory volume in 0.3 s (FEV 0.3), vital capacity (VC), and dynamic lung compliance (Cydn).

2.3.3 Lung tissue pathology:

The left lung was fixed with 10% formaldehyde for 72 hours, embedded in paraffin, sectioned at 4 μm for HE and Masson staining.

Pathological scoring of alveolitis and pulmonary fibrosis was performed according to the Szapiel method, the degree of alveolitis was determined by HE staining, and the degree of pulmonary fibrosis was determined by Masson staining.

The pathological changes in lung tissue were observed using an optical microscope. 6 sections were selected from each group and 6 fields were selected from each section. The degrees of alveolitis and pulmonary fibrosis were scored according to the standards shown in Table 2:

Table 2 Alveolitis and pulmonary fibrosis grading and scoring standards

| Grading | Alveolitis | Pulmonary fibrosis | Score |
|---|---|---|---|
| None | No alveolitis | No pulmonary fibrosis | 0 |
| Mild | Mononuclear cell infiltration and widening of alveolar intervals are limited to local and near-pleural areas, and the area is less than 20% of the whole lung, normal alveolar structure | Pleural and subpleural pulmonary interstitial fibrosis occur with a lesion area less than 20% of the whole lung, and the alveolar structure is disordered | 1 |
| Moderate | The affected area accounts for 20% to 50% of the whole lung, and the area near the pleura is more severe. | Local fibrosis occurs with a lesion area extending from the pleura and accounting for 20% to 50% of the whole lung | 2 |
| Severe | The area of alveolitis is greater than 50%, and consolidation caused by mononuclear cells and hemorrhage | Cavities of varying sizes are visible, and the lesion area is greater than 50% | 3 |
| | in the alveolar space is occasionally seen. | | |

2.3.4 Expression of CoL I and α-SMA in lung tissue:

The detection was performed by Immunohistochemistry.

The lung tissue was sliced into 4 μm sections and baked at 60°C for 3 hours. The paraffin sections were dewaxed in xylene, dehydrated in gradient alcohols, and washed in distilled water for 2 minutes in order. The washed sections were then boiled in 0.01 M citrate buffer in a microwave oven for antigen retrieval. A 3% hydrogen dioxide was added dropwise to the sections to block endogenous peroxidase. After the sections were blocked with a 5% BSA blocking solution and incubated at 37°C for 30 minutes, the primary antibody was added dropwise thereto (the dilution ratio was 1:500 for CoL I,

and 1:2000 for α-SMA), and the sections were incubated overnight in a humid box at 4°C. HRP-labeled goat anti-rabbit secondary antibody was added dropwise, and the sections were incubated at 37°C for 20 minutes; and then SABC was added dropwise and the sections were incubated at 37°C for 30 minutes. Thereafter, a freshly prepared DAB chromogenic solution was added dropwise, and the sections were observed under a microscope, and the color development was stopped once brownish yellow appeared. The sections were counterstained with hematoxylin for 30 seconds, differentiated with a 1% hydrochloric acid in alcohol for 3 seconds; and dehydrated in gradient alcohols in sequence. The sections were dried in a fume hood, sealed with neutral gum, and dried in the air. Images were collected with Olympus-DP70 microscope (Image-Pro Plus 6.0, IPP 6.0), 6 fields were randomly selected for each section, and the image integrated optical density (IOD) was calculated.

**2.4 Statistical methods:**

IBM SPSS22.0 statistical software was used for data analysis. The enumeration data were analyzed using the chi-square test; and for the measurement data, the repeated measurement data of rat weight and lung function were analyzed using repeated measures analysis of variance; the differences among groups were compared using one-way ANOVA, and the least significant difference method was used for homogeneous variances, and Dunnett's T3 method was used for uneven variances, the significance level was $\alpha$=0.05, and the data were expressed as mean ( $\overline{X}$) ± standard error (SE).

**2.5 R value comprehensive evaluation:**

The R value comprehensive evaluation method (Copyright No.: Guozuodengzi-2013-A-00096833) was used to comprehensively evaluate a total of 8 indicators including rat lung function (forced expiratory vital capacity (FVC), forced expiratory volume in 0.3 seconds (FEV0.3), deep inspiratory volume (IC), and dynamic lung compliance (Cydn)), lung tissue pathology (HE staining, MASSON staining), and lung tissue immunohistochemistry (CoL I and α-SMA).

The data were standardized and the $R_{comprehensive}$ value data set was established ($R_{comprehensive}$ = R-(-2)). After statistical analysis, the comprehensive corrective effect $R_{comprehensive}$ was finally obtained. When $R_{comprehensive}$ <1, it means that the comprehensive corrective degree of the drug on such indicators exceeds the normal value. When $R_{comprehensive}$ = 1, it means that the comprehensive corrective degree of the drug on such indicators achieves the best effect. When 1< $R_{comprehensive}$ <2, it means that the drug has a certain corrective effect on such indicators. When $R_{comprehensive}$ = 2, it means that the drug has no corrective effect on such indicators. When $R_{comprehensive}$ > 2, it means that the drug has a reverse exacerbating effect on such indicators. According to value of $R_{comprehensive}$, the comprehensive effects of the drug on the indicators were ranked.

**3 Formula screening**

Screening was conducted based on the traditional Chinese medicine component formula in CN109394776B and optimized the formula to detect the therapeutic effect of the simplified formulas or the formulas obtained by reducing dose.

It was first confirmed through preliminary optimization experiments that the formulas obtained by not using or using less of some components of the traditional Chinese medicine component formula in CN109394776B (for example, ginsenoside and/or peimine) are still effective in treating diffuse interstitial pulmonary fibrosis in rats, and the R value comprehensive evaluation method was used to confirm that some formulas had a better effect in corrective degree.

On the basis of the above experiments, new formulas obtained by combining individual components of the traditional Chinese medicine component formula in CN109394776B were used to treat grouped rats according to dose. The specific formulas and doses are shown in Table 3. Among them, formula 1 is the traditional Chinese medicine component formula in CN109394776B.

Table 3 Experimental grouping and Drug combination

| Form ula | Dose (mg/kg) | ginsenoside Re /mg | icarii n/mg | isoliquiritigenin /mg | nobilet in /mg | peimi ne /mg | paeoniflo rin /mg |
|---|---|---|---|---|---|---|---|
| 1 | 8.04 | 3.08 | 3.85 | 0.31 | 0.08 | 0.48 | 0.24 |
| 2 | 4.72 | 0 | 3.85 | 0.31 | 0.08 | 0.24 | 0.24 |
| 3 | 3.85 | 0 | 3.85 | 0 | 0 | 0 | 0 |
| 4 | 0.31 | 0 | 0 | 0.31 | 0 | 0 | 0 |
| 5 | 0.08 | 0 | 0 | 0 | 0.08 | 0 | 0 |
| 6 | 0.24 | 0 | 0 | 0 | 0 | 0.24 | 0 |

(continued)

| Form ula | Dose (mg/kg) | ginsenoside Re /mg | icarii n/mg | isoliquiritigenin /mg | nobilet in /mg | peimi ne /mg | paeoniflo rin /mg |
|---|---|---|---|---|---|---|---|
| 7 | 0.24 | 0 | 0 | 0 | 0 | 0 | 0.24 |
| 8 | 0.55 | 0 | 0 | 0.31 | 0 | 0 | 0.24 |
| 9 | 4.40 | 0 | 3.85 | 0.31 | 0 | 0 | 0.24 |
| 10 | 0.63 | 0 | 0 | 0.31 | 0.08 | 0 | 0.24 |
| 11 | 0.79 | 0 | 0 | 0.31 | 0 | 0.24 | 0.24 |
| 12 | 0.87 | 0 | 0 | 0.31 | 0.08 | 0.24 | 0.24 |
| 13 | 4.48 | 0 | 3.85 | 0.31 | 0.08 | 0 | 0.24 |
| 14 | 4.64 | 0 | 3.85 | 0.31 | 0 | 0.24 | 0.24 |

The respective ingredients were provided and identified by the Drug Analysis Laboratory, School of Pharmacy, Henan University of Chinese Medicine. Before use, they were prepared into a suspension by being dissolved in a 0.5% sodium carboxymethyl cellulose (CMC-Na).

Table 4 Grouping and treatment

| Group | Intragastric dose (mg/kg/d) | Drug |
|---|---|---|
| Blank | 2ml/time | saline |
| Model | 2ml/time | saline |
| Pirfenidone | 108 | Pirfenidone solution |
| Formula 1 | 8.04 | icariin +isoliquiritigenin +peimine +nobiletin+paeoniflorin +ginsenoside Re |
| Formula 2 | 4.72 | icariin +isoliquiritigenin +peimine +nobiletin+paeoniflorin |
| Formula 3 | 3.85 | icariin |
| Formula 4 | 0.31 | isoliquiritigenin |
| Formula 5 | 0.08 | nobiletin |
| Formula 6 | 0.24 | peimine |
| Formula 7 | 0.24 | paeoniflorin |
| Formula 8 | 0.55 | isoliquiritigenin +paeoniflorin |
| Formula 9 | 4.40 | icariin +paeoniflorin +isoliquiritigenin |
| Formula 10 | 0.63 | nobiletin+paeoniflorin +isoliquiritigenin |
| Formula 11 | 0.79 | peimine +paeoniflorin +isoliquiritigenin |
| Formula 12 | 0.87 | nobiletin+peimine +paeoniflorin +isoliquiritigenin |
| Formula 13 | 4.48 | icariin +nobiletin+paeoniflorin +isoliquiritigenin |
| Formula 14 | 4.64 | icariin +peimine +paeoniflorin +isoliquiritigenin |

**4 Experimental results**

**4.1 General conditions**

The rats in the blank group moved freely, responded quickly, had smooth and shiny fur, and had normal food and water intake.

On the first day of modeling, the rats in the model group had significantly reduced activity, mental fatigue, sleepiness, and significantly reduced food and water intake, but no deaths occurred. In the first week after modeling, the rats had bleeding around the eyes, mouth, nose, and claw nails, visible blood scabs, and recovered food and water intake, but not recovered to normal level. In the 2nd week after modeling, the rats in the model group showed no bleeding, their food and water intake returned to normal level, their fur was white and dull, and some rats developed sneezing symptoms. In the 3rd to 4th week after modeling, the rats in the model group moved freely and had normal food and water intake.

In the 5th to 6th week after administration, the pirfenidone group and respective formula groups showed improvements in above symptoms to varying degrees.

### 4.2 Lung function

The lung function monitoring results are shown in Table 5. Compared with the blank group, FVC, FEV0.3, IC, and Cydn in the model group were significantly reduced (*P*<0.01, *P*<0.05).

Compared with the model group, the FVCs of groups of formulas 7, 8, and 11 were significantly increased (*P*<0.01, *P*<0.05), the FEV0.3s of groups of formulas 1, 2, 7, 8, and 11 were significantly increased (*P*<0.01, *P*<0.05), the ICs of the pirfenidone group and groups of formulas 1, 7, and 8 were significantly increased (*P*<0.01, *P*<0.05), and the Cydns of groups of formulas 5 and 13 were increased (*P*<0.05). Compared with the pirfenidone group, the FVC and FEV0.3 of groups of formulas 7 and 8 were both increased (*P*<0.05).

Table 5 Lung function related indicators ( $\overline{X}\pm$SE)

| Group | FVC (ml/s) | FEV0.3 (ml/s) | IC (ml/s) | Cydn (ml/s) |
|---|---|---|---|---|
| Blank | 14.32+0.79 | 13.77+0.76 | 11.47+0.62 | 0.38±0.03 |
| Model | 10.11±0.87** | 9.51±0.83** | 7.65+0.71** | 0.26±0.02* |
| Pirfenidone | 11.35±1.09 | 10.68+0.97 | 10.69±0.98$^{\#}$ | 0.33+0.03 |
| Formula 1 | 12.49+0.36 | 13.13±0.39$^{\#\#}$ | 11.94±0.44$^{\#\#}$ | 0.34±0.02 |
| Formula 2 | 11.33+0.96 | 10.35±0.83$^{\#}$ | 8.30+0.72 | 0.30+0.03 |
| Formula 3 | 11.68±1.50 | 10.46±1.58 | 8.48+0.91 | 0.34±0.05 |
| Formula 4 | 11.52±1.37 | 10.42±1.43 | 8.83+0.92 | 0.30+0.03 |
| Formula 5 | 12.77±1.39 | 11.44±1.41 | 9.77+0.62 | 0.35±0.02$^{\#}$ |
| Formula 6 | 12.98±1.34 | 12.04±1.36 | 8.48+0.86 | 0.30+0.03 |
| Formula 7 | 15.95±1.36$^{\#\#\triangle}$ | 14.70±1.25$^{\#\#\triangle}$ | 9.84±0.75$^{\#}$ | 0.31±0.03 |
| Formula 8 | 16.14±0.79$^{\#\#\triangle}$ | 15.03±0.70$^{\#\#\triangle}$ | 10.28±0.64$^{\#}$ | 0.34±0.02 |
| Formula 9 | 13.12±1.28 | 11.87±1.52 | 8.39+0.80 | 0.26±0.03 |
| Formula 10 | 11.88+0.94 | 11.23+0.97 | 8.06+0.97 | 0.28±0.04 |
| Formula 11 | 14.44±1.12$^{\#\#}$ | 13.16±1.04$^{\#}$ | 9.12+0.73 | 0.31±0.03 |
| Formula 12 | 12.14±1.20 | 11.22±1.16 | 9.18+0.84 | 0.33+0.04 |
| Formula 13 | 12.56±1.26 | 10.84±1.35 | 9.64+0.73 | 0.37±0.04$^{\#}$ |
| Formula 14 | 11.25±0.49 | 10.78+0.48 | 8.55+0.64 | 0.33+0.02 |

Note 1: N=10, * indicates *P*<0.05, ** indicates *P*<0.01, compared with the blank group; $^{\#}$ indicates *P*<0.05, $^{\#\#}$ indicates *P*<0.01, compared with the model group; $^{\triangle}$ indicates *P*< 0.05, $^{\triangle\triangle}$ indicates *P*<0.01, compared with the pirfenidone group.

### 4.3 Lung tissue pathology

The alveolar structure of the rats in the blank group was intact, and the alveolar intervals were not thickened. For the rats in the model group, the alveolar walls were broken and fused, the alveolar cavities of different sizes were visible, the alveolar structure was severely damaged, the alveolar walls were widened, and there were a large number of inflammatory cells infiltrated, collagen deposition increased, and fibrotic scars formed. There were improvements in the above pathological damages to varying degrees in the rats of pirfenidone group and respective formula groups.

The scoring results based on the H&E and Masson staining images of rat lung tissue are shown in Table 6, and the H&E staining images are shown in Figs. 1-17, and the Masson staining images are shown in Figs. 18-34. Compared with the blank group, the alveolitis and pulmonary fibrosis scores in the model group were significantly increased (*P*<0.01). Compared with the model group, the alveolitis scores of groups of formulas 3 and 6 were significantly reduced (*P*<0.01, *P*<0.05); the pulmonary fibrosis scores of groups of formulas 7, 8, 10, 13, and 14 were significantly reduced (*P*<0.01, *P*<0.05).

**Table 6 Alveolitis and pulmonary fibrosis scores ( $\overline{X}\pm$SE)**

| Group | N | Alveolitis (score) | Pulmonary fibrosis (score) |
|---|---|---|---|
| Blank | 6 | 0.33+0.21 | 0.00+0.00 |
| Model | 6 | 2.00±0.37** | 2.00+0.37** |
| Pirfenidone | 6 | 1.33+0.24 | 1.33+0.37 |
| Formula 1 | 6 | 1.67+0.33 | 1.83+0.40 |

(continued)

| Group | N | Alveolitis (score) | Pulmonary fibrosis (score) |
|---|---|---|---|
| Formula 2 | 6 | 1.61±0.30 | 1.39+0.38 |
| Formula 3 | 6 | 0.94±0.39## | 1.33+0.31 |
| Formula 4 | 6 | 1.72+0.25 | 1.78+0.33 |
| Formula 5 | 6 | 1.39+0.54 | 1.56+0.49 |
| Formula 6 | 6 | 1.17±0.19# | 1.39+0.23 |
| Formula 7 | 6 | 1.44+0.27 | 0.94±0.16## |
| Formula 8 | 6 | 1.39+0.22 | 1.17±0.32# |
| Formula 9 | 6 | 1.39+0.16 | 1.33+0.24 |
| Formula 10 | 6 | 1.33+0.21 | 0.83±0.24## |
| Formula 11 | 6 | 1.50+0.35 | 1.39+0.37 |
| Formula 12 | 6 | 1.56+0.25 | 1.44+0.38 |
| Formula 13 | 6 | 1.72+0.32 | 1.17±0.33# |
| Formula 14 | 6 | 1.39+0.23 | 1.22±0.28# |

Note: * indicates *P*<0.05, ** indicates *P*<0.01, compared with the blank group; # indicates *P*<0.05, ## indicates *P*<0.01, compared with the model group.

**4.4 CoL I and α-SMA protein expression changes in lung tissue**

Based on the results of CoL I and α-SMA staining images of rat lung tissue, the changes in CoL I and α-SMA protein expression in lung tissue are shown in Table 7. The CoL I staining images are shown in Figs. 35-51. The α-SMA staining images are shown in Figs. 52-68. Compared with the blank group, the expression of CoL I and α-SMA proteins in the lung tissue of rats in the model group was significantly increased (*P*<0.01, *P*<0.05). Compared with the model group, the expression of CoL I protein in the pirfenidone group, and the groups of formulas 1, 2, 4, 7, 8, 9, 11, 12, and 14 was significantly reduced (*P*<0.01, *P*<0.05); the expression of α-SMA protein in the groups of formula 2, 5, 6, and 7 was significantly reduced (*P*<0.05).

**Table 7 Changes of COL I and α-SMA in respective groups ( $\overline{X}$±SE)**

| Group | COL I (IOD) | α-SMA (IOD) |
|---|---|---|
| Blank | 9.33+0.80 | 9.17+0.65 |
| Model | 19.93±2.13** | 12.30±0.43* |
| Pirfenidone | 10.41±0.63## | 12.57±1.17 |
| Formula 1 | 13.69±0.93## | 11.69+0.67 |
| Formula 2 | 15.24±1.70# | 10.39±0.89# |
| Formula 3 | 17.80±1.44 | 12.03±1.87 |
| Formula 4 | 15.54±2.59# | 12.20+0.89 |
| Formula 5 | 18.40+0.89 | 9.030±0.8# |
| Formula 6 | 17.37+0.89 | 9.647±0.8# |
| Formula 7 | 13.61±1.90## | 10.57±0.36# |
| Formula 8 | 8.157±0.3## | 11.25±0.36 |
| Formula 9 | 6.765±0.3## | 12.56±0.36 |
| Formula 10 | 17.06+0.71 | 12.70±1.63 |
| Formula 11 | 12.20±1.41## | 13.86±1.21 |
| Formula 12 | 12.04±1.88## | 11.70±1.08 |
| Formula 13 | 19.52±1.08 | 12.79±1.08 |
| Formula 14 | 13.46±1.50## | 11.78+0.95 |

The same as Note 1. N=6.

### 4.6 R value Comprehensive evaluation

A total of 8 indicators, i.e., lung function (FVC, FEV0.3, IC, Cydn), lung tissue pathology (H&E staining, Masson staining), and collagen deposition (COL I, α-SMA) were calculated using the R value comprehensive evaluation method. The results are shown in Table 8.

The results show that the respective treatment groups had a significant corrective effect on PF rats (*P*<0.01). The correction degree is as follows: Formula 8> Formula 1> Formula 7> Formula 5> Formula 6> Pirfenidone> Formula 11> Formula 9> Formula 14> Formula 12> Formula 13> Formula 2> Formula 3> Formula 10> Formula 4. Except for the groups of formulas 3, 4, 10, and 13, the respective treatment groups had significant improvement compared with the model group (*P*<0.01, *P*<0.05). The Formula 7 (paeoniflorin) was better than the Formula 3 (icariin), the Formula 4 (isoliquiritigenin), and the Formula 5 (nobiletin) (*P*<0.05); and the Formula 8 (Isoliquiritigenin + Paeoniflorin) was better than pirfenidone, Formulas 2, 3, 4, and 9-14.

**Table 8 Total efficacy related indicator R value ( $\overline{X}$±SE)**

| Group | N | Total efficacy related indicator R value |
|---|---|---|
| Model | 8 | 2.00+0.00 |
| Pirfenidone | 8 | 1.61+0.32$^{\#}$ |
| Formula 1 | 8 | 1.38+0.46## |
| Formula 2 | 8 | 1.69+0.12$^{\#\circ}$ |
| Formula 3 | 8 | 1.76+0.16° |
| Formula 4 | 8 | 1.82±0.11°° |
| Formula 5 | 8 | 1.48±0.46$^{\#\#}$ |
| Formula 6 | 8 | 1.57±0.29$^{\#\#}$ |
| Formula 7 | 8 | 1.41±0.27$^{\#\#}$□■◇ |
| Formula 8 | 8 | 31.22±0.47$^{\#\#}$△□□■■◇◇ |
| Formula 9 | 8 | 1.63±0.44$^{\#}$★★ |
| Formula 10 | 8 | 1.76±0.17★★ |
| Formula 11 | 8 | 1.62±0.37$^{\#}$★★ |
| Formula 12 | 8 | 1.68±0.15$^{\#}$★★ |
| Formula 13 | 8 | 1.72±0.28★★ |
| Formula 14 | 8 | 1.64±0.19$^{\#}$★★ |

Note: * indicates *P*<0.05, ** indicates *P*<0.01, compared with the blank group; # indicates *P*<0.05, ## indicates P<0.01, compared with the model group; △ indicates *P*<0.05, compared with the pirfenidone group; ° indicates *P*<0.05, °° indicates *P*<0.01, compared with formula 1; □ indicates *P*<0.05, □□ indicates *P*<0.01, compared with formula 2; ■ indicates *P*<0.05, compared with formula 3; ◇ indicates *P*<0.05, ◇ ◇ indicates *P*<0.01, compared with formula 4; ** indicates *P*<0.01, compared with formula 8.

### 5. Conclusion

The respective formulas can improve to varying degrees the lung function and pulmonary fibrosis and reduce the inflammatory response and collagen deposition in lung tissue in rats with pulmonary fibrosis. Among them, the formula 8 (isoliquiritigenin combined with paeoniflorin) is more effective and more economical.

### Example 2 Evaluation of efficacy of ingredients of the formula 8

In order to evaluate the difference in the efficacy of isoliquiritigenin and paeoniflorin alone and in combination, a study was conducted by splitting the Formula 8 into isoliquiritigenin, paeoniflorin and combination thereof, each adopting the dose of 0.55 mg/kg/d.

### 1. Experimental materials

The experimental materials and research methods were the same as those in Example 1.

**2. Treatment**

A total of 72 rats were randomly divided into blank group, model group, isoliquiritigenin group, paeoniflorin group, combination group, and pirfenidone group, 6 groups in total, 12 rats in each group, and 6 female /group, 6 male /group. The modeling day was set as day 0. From the 29th day, the blank group (normal rats without modeling) and the model group were administered intragastrically with physiological saline (2ml/rat), the isoliquiritigenin group was administered with isoliquiritigenin (0.55 mg/kg/d), the paeoniflorin group was administered with paeoniflorin (0.55 mg/kg/d), and the combination group was administered with isoliquiritigenin + paeoniflorin (0.55 mg/kg/d), once daily, for 14 days, and the samples were collected at the end of the $42^{nd}$ day.

**3. Indicator detection**

3.1 General conditions:

The death, bleeding around the eyes, bleeding of mouth and nose of the rats were observed. The rats' fur color, mental status, eating and activities were observed every day.

3.2 Lung function:

Before collecting the sample, the rats were anesthetized and submitted to a tracheotomy, and the tracheal tube was inserted into the rat's trachea and fixed. The FinePointe™ series PFT measurement system was used to detect the rat's forced vital capacity (FVC), forced expiratory volume in 0.3 s (FEV 0.3), vital capacity (VC), and dynamic lung compliance (Cydn).

3.3 Lung tissue pathology:

The left lung was fixed with 10% formaldehyde for 72 hours, embedded in paraffin, sectioned at 4 μm for HE and Masson staining.

Pathological scoring of alveolitis and pulmonary fibrosis was performed according to the Szapiel method, the degree of alveolitis was determined by HE staining, and the degree of pulmonary fibrosis was determined by Masson staining.

The pathological changes in the lung tissue were observed using an optical microscope. 6 sections were selected from each group and 6 fields were selected from each section. The degrees of alveolitis and pulmonary fibrosis were scored according to the standards shown in Table 2 in Example 1:

**4. Results**

**4.1 Lung function**

The pulmonary function monitoring results are shown in Table 9.

Compared with the blank group, the FVC, FEV0.3, IC, and Cydn in the model group were significantly reduced ($P$<0.01, $P$<0.05). Compared with the model group, the FEV0.3 of the isoliquiritigenin group was increased, the FVC and FEV0.3 of the paeoniflorin group were increased, the FVC, FEV0.3, IC, and Cydn of the combination group were all increased significantly, and the FEV0.3, IC of the pirfenidone group were increased ($P$<0.01, $P$<0.05). The combination group improved FVC better than the pirfenidone group, improved FVC and FEV0.3 better than the isoliquiritigenin group, and improved FEV0.3 better than the paeoniflorin group ($P$<0.05).

**Table 9 Lung function changes in respective groups ( $\bar{X}\pm$SE)**

| Group | FVC (ml/s) | FEV0.3 (ml/s) | IC (ml/s) | Cydn (ml/s) |
|---|---|---|---|---|
| Blank | 15.42+0.67 | 14.03+0.65 | 11.78+0.69 | 0.42±0.05 |
| Model | 10.78+0.69** | 9.87+0.79** | 8.04+0.69** | 0.25+0.03* |
| Isoliquiritigenin | 11.34+0.78 | 11.09±1.01$^{\#}$ | 9.03+0.88 | 0.32±0.04 |
| Paeoniflorin | 13.49±1.17$^{\#}$ | 12.88±0.98$^{\#}$ | 9.62+0.54 | 0.31+0.54 |
| Combination | 14.79±0.85$^{\#\#\triangle\diamond}$ | 14.76±1.07$^{\#\#\diamond\blacktriangle}$ | 11.08±0.66$^{\#}$ | 0.37±0.02$^{\#}$ |

(continued)

| Group | FVC (ml/s) | FEV0.3 (ml/s) | IC (ml/s) | Cydn (ml/s) |
|---|---|---|---|---|
| Pirfenidone | 12.34±1.11 | 13.46±1.04# | 10.77±0.83# | 0.33+0.03 |
| Note 1: N=10, * indicates *P*<0.05, ** indicates *P*<0.01, compared with the blank group; # indicates *P*<0.05, ## indicates P<0.01, compared with the model group; △ indicates *P*< 0.05, △△ indicates *P*<0.01, compared with the pirfenidone group; ◇ indicates *P*<0.05, ◇◇ indicates *P*<0.01, compared with the isoliquiritigenin group; ▲ indicates *P*<0.05, ▲▲ indicates *P*<0.01, compared with the paeoniflorin group. | | | | |

### 4.2 Lung tissue pathology

The scoring results based on H&E and Masson staining images of rat lung tissue are shown in Table 10, and the H&E staining images are shown in Figs. 69-74, and the Masson staining images are shown in Figs. 75-80.

Compared with the blank group, the degree of alveolitis and pulmonary fibrosis in the model group was significant (*P*<0.01). Compared with the model group, the isoliquiritigenin and paeoniflorin group could reduce the pulmonary fibrosis score (*P*<0.05), and the alveolitis and pulmonary fibrosis scores of the combination group and the pirfenidone group were significantly reduced (*P*<0.01, *P*<0.05). The combination group was better than the isoliquiritigenin group in reducing alveolitis and pulmonary fibrosis scores, and was better than the paeoniflorin group in reducing pulmonary fibrosis score (*P*<0.05).

**Table 10 Lung pathology score ( $\overline{X}$±SE)**

| Group | N | Alveolitis (score) | Pulmonary fibrosis (score) |
|---|---|---|---|
| Blank | **6** | 0.00+0.00 | 0.00+0.00 |
| Model | **6** | 2.11±0.23## | 2.36±0.32## |
| Isoliquiritigenin | **6** | 1.68+0.30 | 1.74±0.31# |
| Paeoniflorin | **6** | 1.52+0.29 | 1.65±0.19# |
| Combination | **6** | 1.39±0.22#◇ | 1.40±0.25##◇▲ |
| Pirfenidone | **6** | 1.43±0.28# | 1.46±0.39##◇ |
| **Note: Same as Table 9.** | | | |

### 4. Conclusion

In summary, the Formula 8 has good efficacy in treating pulmonary fibrosis. It can relieve symptoms, improve lung function, improve the degree of alveolitis and pulmonary fibrosis in lung tissue, and reduce collagen deposition in lung tissue, and can effectively alleviate the process of pulmonary fibrosis. Further, the Formula 8 has the advantages of clear ingredients, small dosage form, and portability, etc. After further evaluating the effects of the Formula 8, it is found that even if the single ingredients in Formula 8 are used in the same amount as the Formula 8, the effects of the Formula 8 cannot be achieved. Therefore, the Formula 8 (isoliquiritigenin + paeoniflorin) is a novel drug for the long-term treatment of pulmonary fibrosis and has high practical value.

The above-described examples are only preferred examples of the present invention and are not intended to limit the present invention in any form. Although the present invention has been disclosed above in preferred examples, they are not intended to limit the present invention. Any person skilled in the art, without departing from the scope of the technical solution of the present invention, can make some changes or modifications to the above disclosed technical contents to obtain equivalent embodiments with equivalent changes, and without departing from the technical solution of the present invention, any simple modifications, equivalent changes and modifications made to the above examples based on technical essence of the present invention are within the scope of the technical solution of the present invention.

## Claims

1. A pharmaceutical composition consisting of paeoniflorin and isoliquiritigenin.

2. The pharmaceutical composition of claim 1, wherein, the weight ratio of paeoniflorin and isoliquiritigenin is 10:90 to 90:10, such as 20:80 to 80:20, 30:70 to 70:30, 40:60 to 60:40, 40:60 to 55:45, or 40:60 to 50:50.

3. The pharmaceutical composition of claim 1, wherein, the weight percentage of paeoniflorin is 10% or more, 20% or more, 25% or more, 30% or more, 35% or more, or 40% or more, and 90% or less, 80% or less, 75% or less, 70% or less, 65% or less, 60% or less, 55% or less, or 50% or less, such as 40% to 50%.

4. The pharmaceutical composition of claim 1, wherein, the paeoniflorin and isoliquiritigenin have a purity of not less than 98% as determined by high performance liquid chromatography.

5. Application of the pharmaceutical composition of anyone of claims 1 to 4 for preparing a medicine for preventing or treating diffuse interstitial pulmonary fibrosis.

6. A medicine for preventing or treating diffuse interstitial pulmonary fibrosis, which contains the pharmaceutical composition of anyone of claims 1 to 4 and does not contain ginsenoside Re, icariin, nobiletin, and peimine.

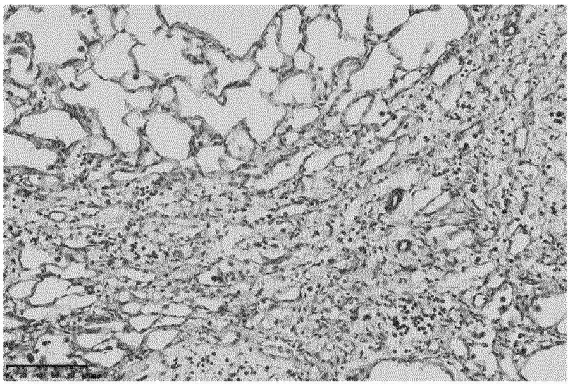

Fig. 1

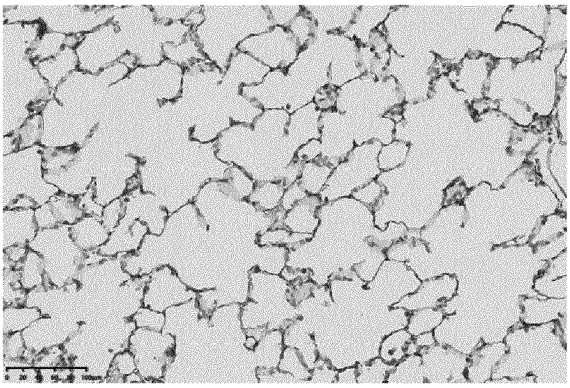

Fig. 2

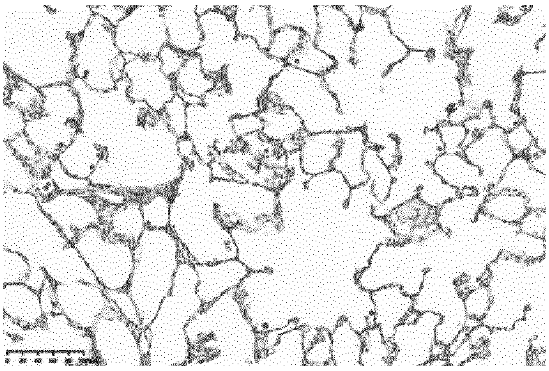

Fig. 3

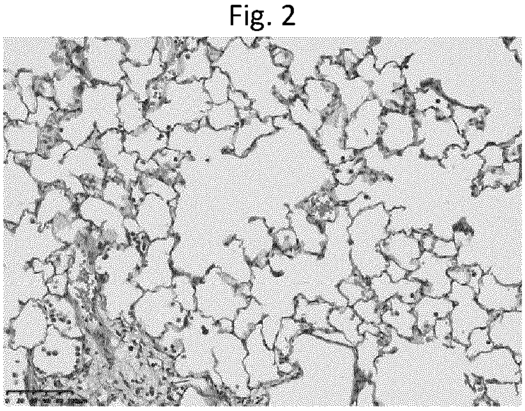

Fig. 4

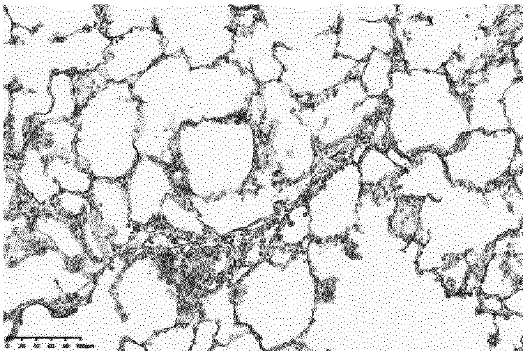

Fig. 5

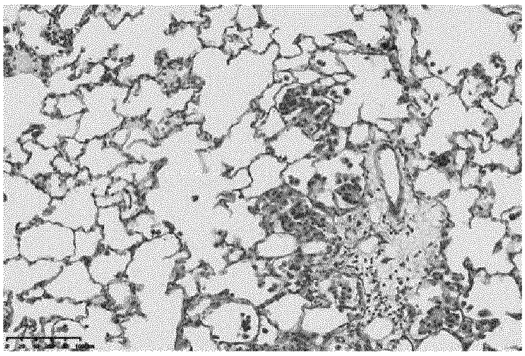

Fig. 6

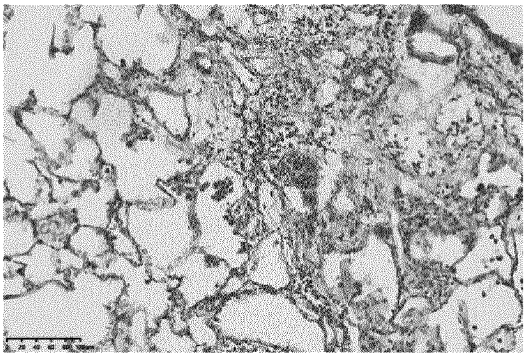

Fig. 7

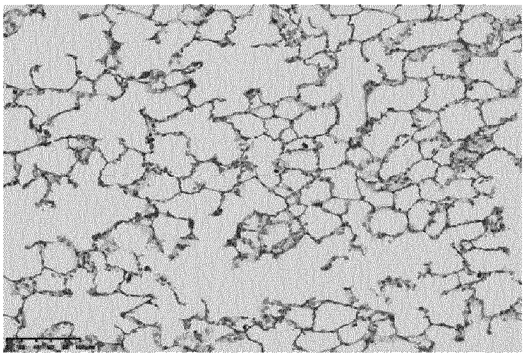

Fig. 8

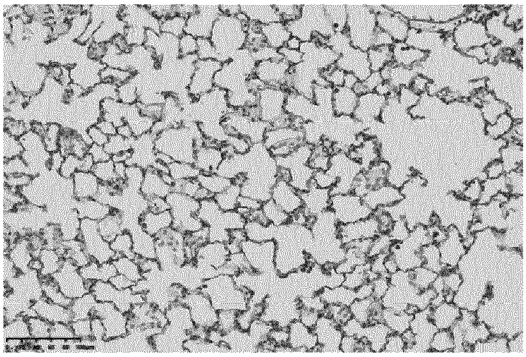

Fig. 9

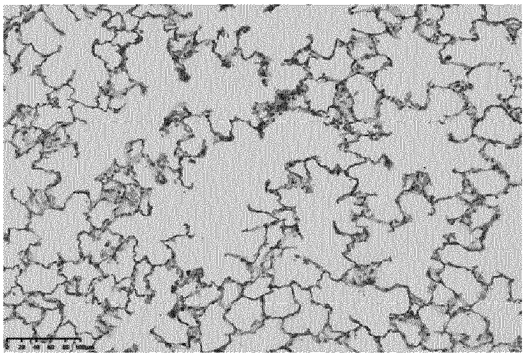

Fig. 10

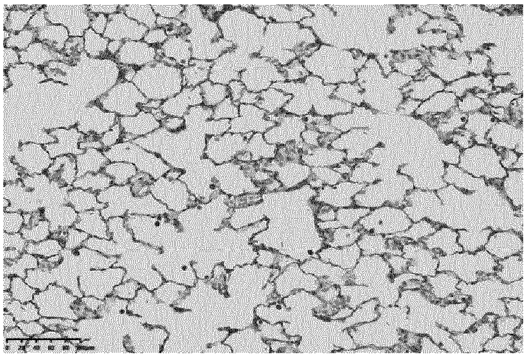

Fig. 11

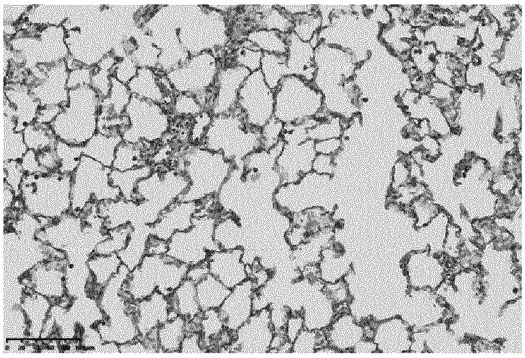

Fig. 12

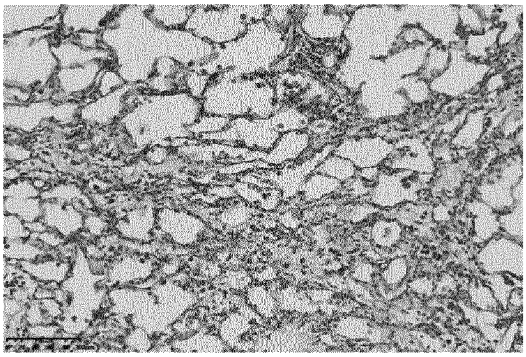
Fig. 13

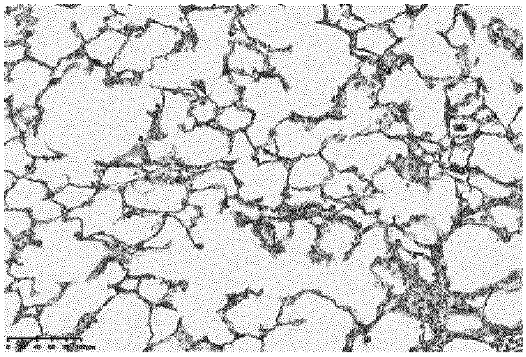
Fig. 14

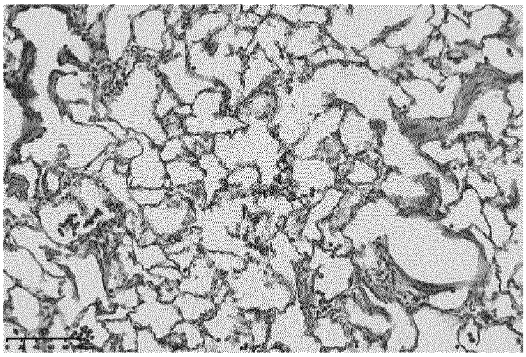
Fig. 15

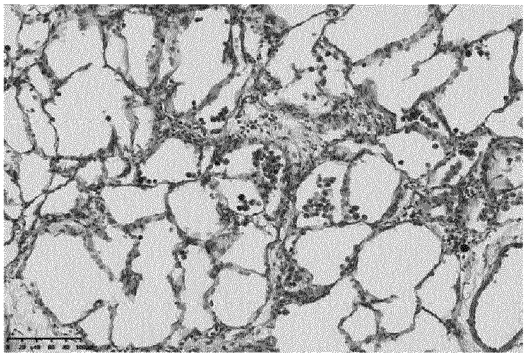
Fig. 16

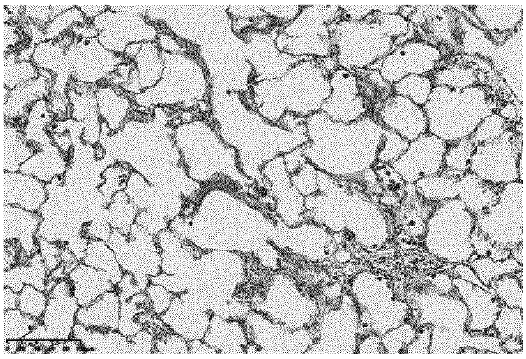
Fig. 17

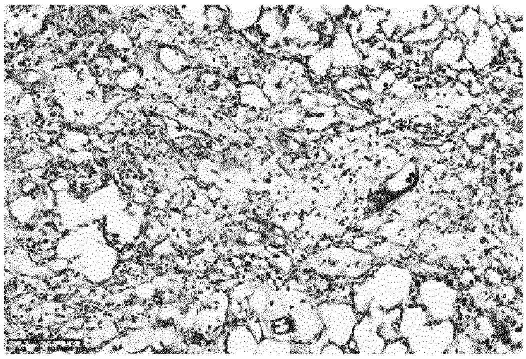
Fig. 18

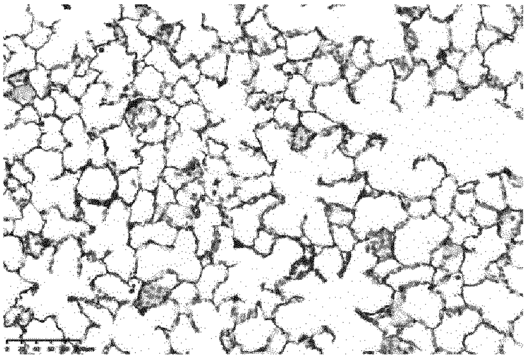

Fig. 19

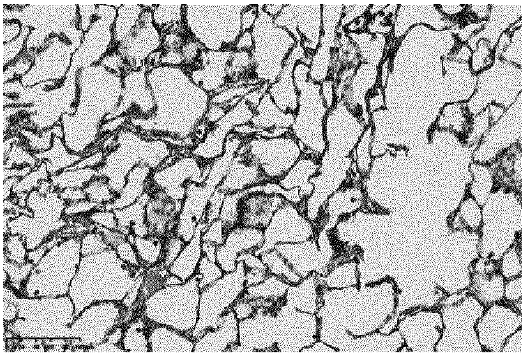

Fig. 20

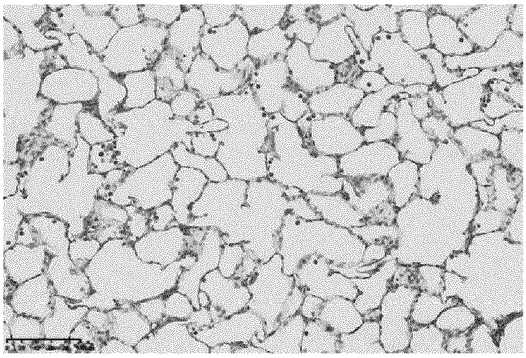

Fig. 21

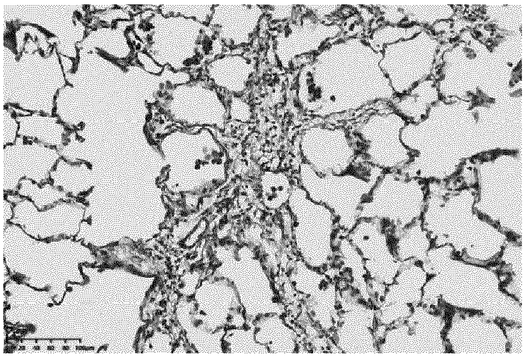

Fig. 22

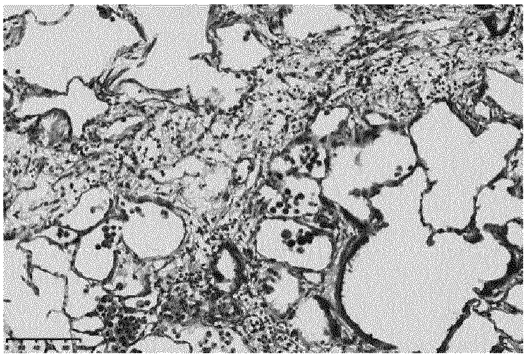

Fig. 23

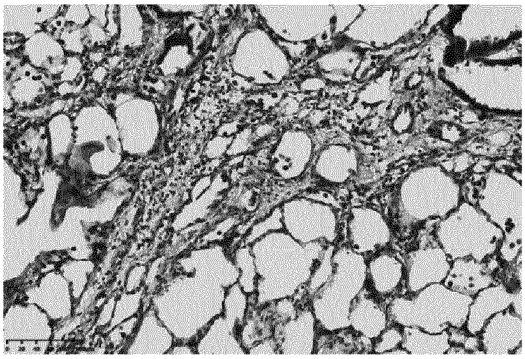

Fig. 24

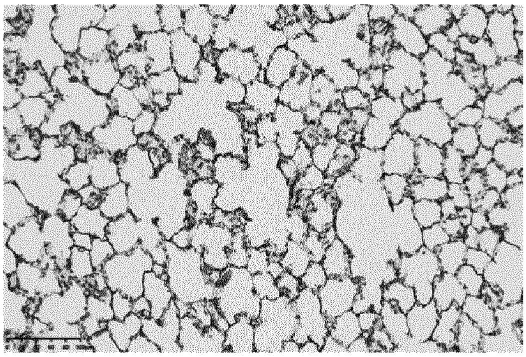
Fig. 25

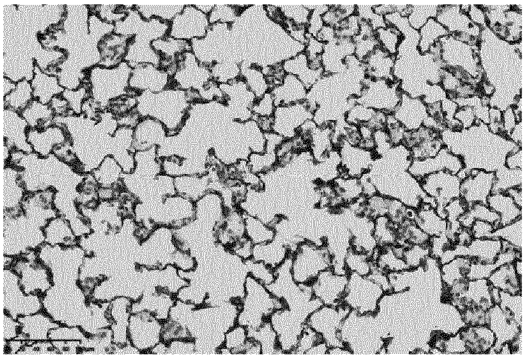
Fig. 26

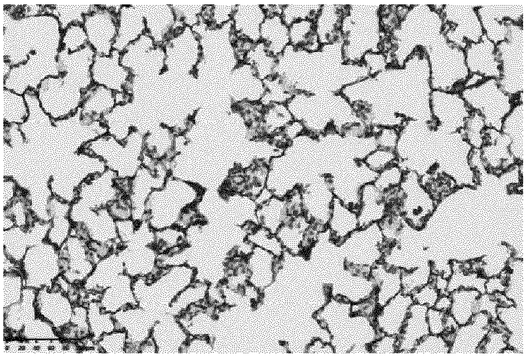
Fig. 27

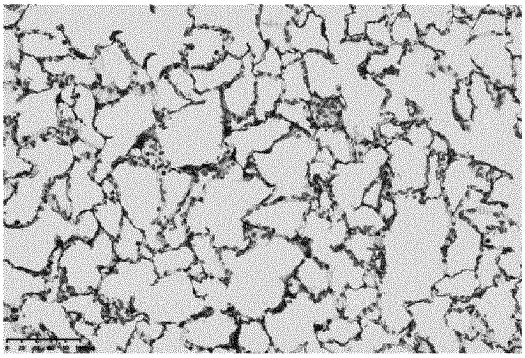
Fig. 28

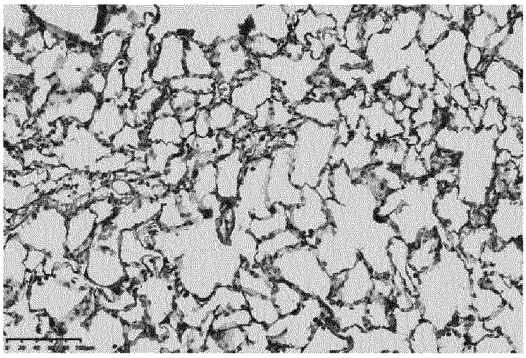
Fig. 29

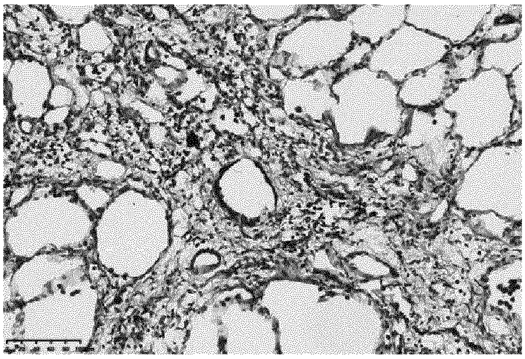
Fig. 30

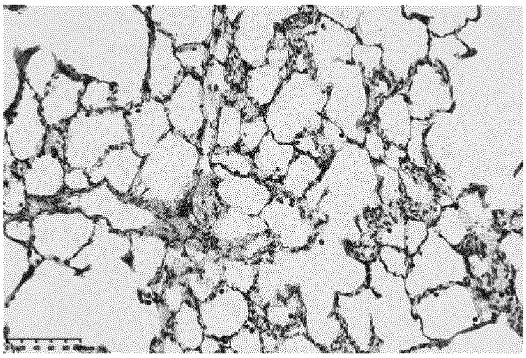

Fig. 31

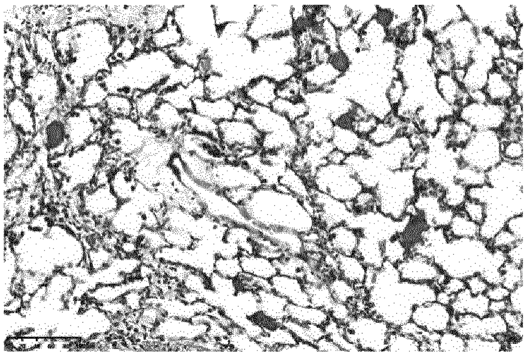

Fig. 32

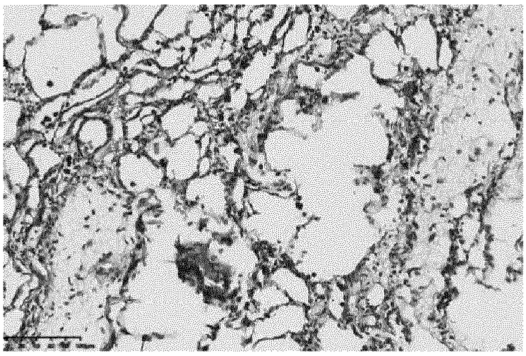

Fig. 33

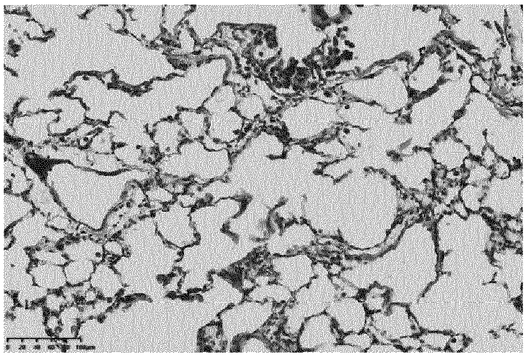

Fig. 34

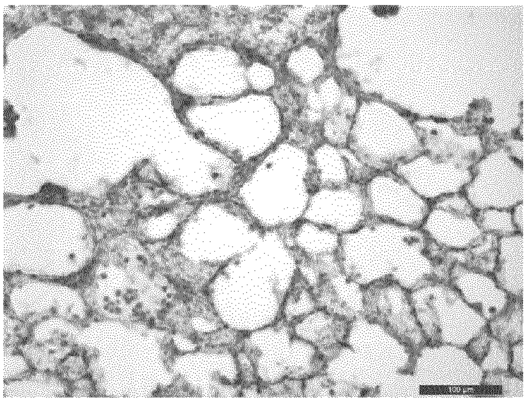

Fig. 35

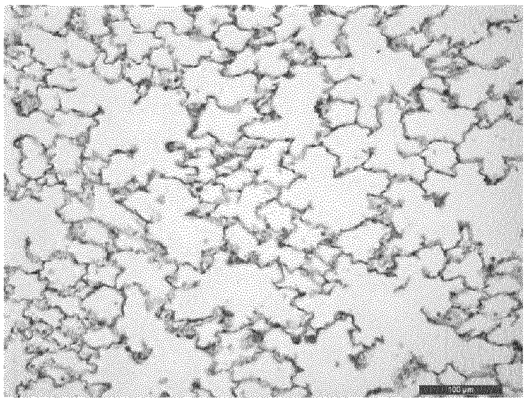

Fig. 36

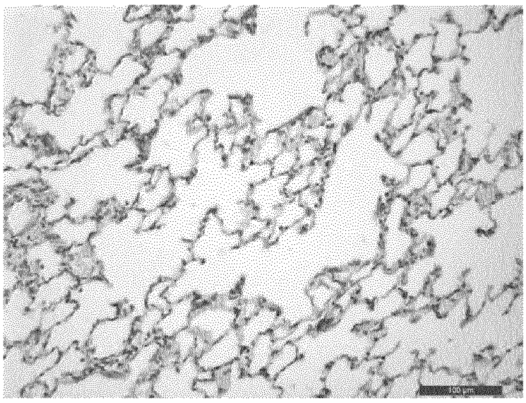

Fig. 37

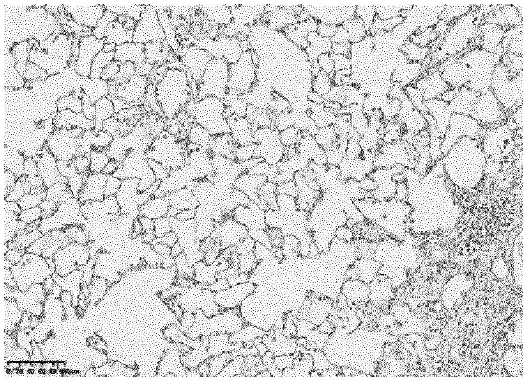

Fig. 38

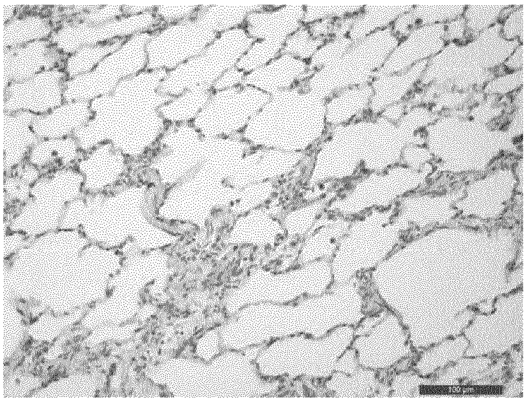

Fig. 39

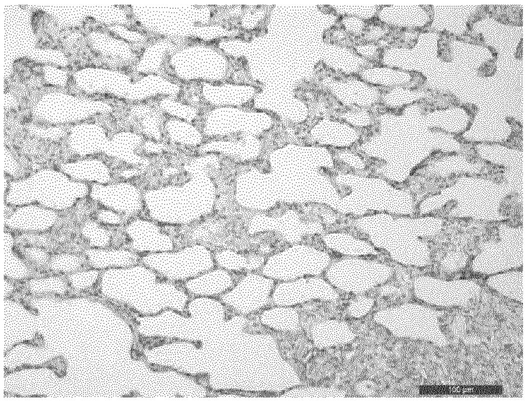

Fig. 40

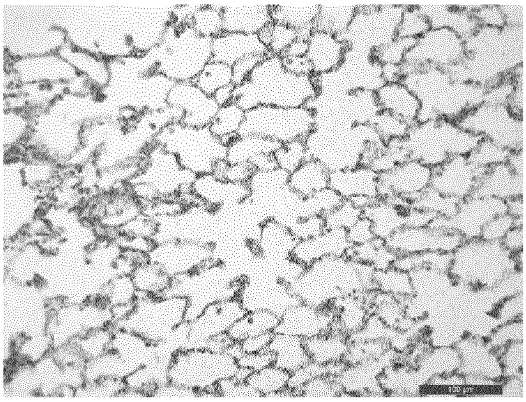

Fig. 41

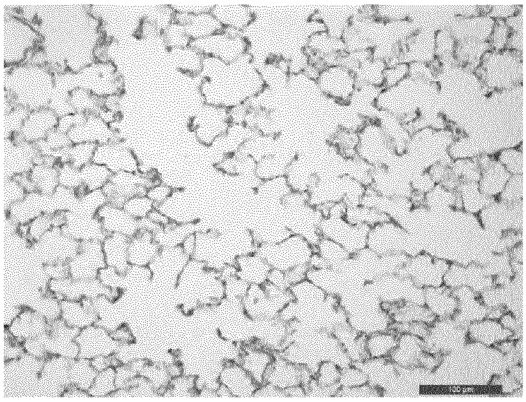

Fig. 42

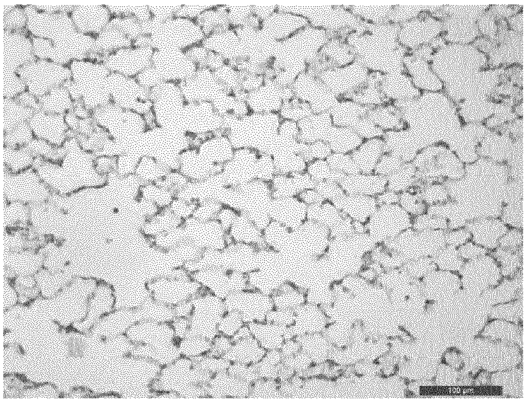
Fig. 43

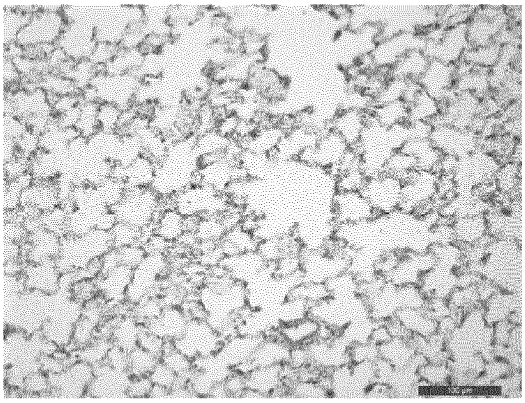
Fig. 44

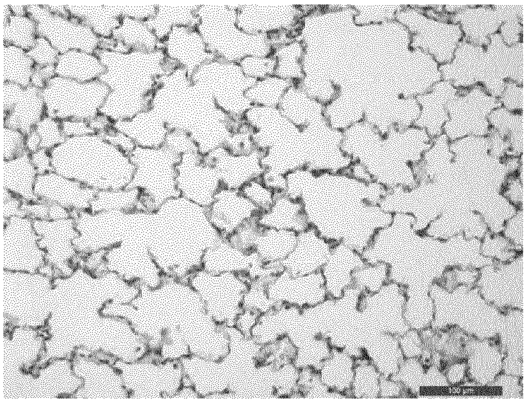
Fig. 45

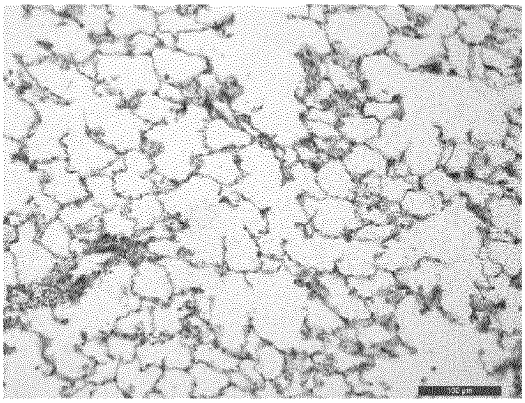
Fig. 46

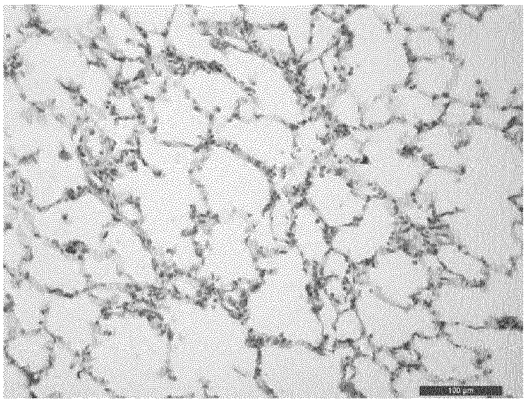
Fig. 47

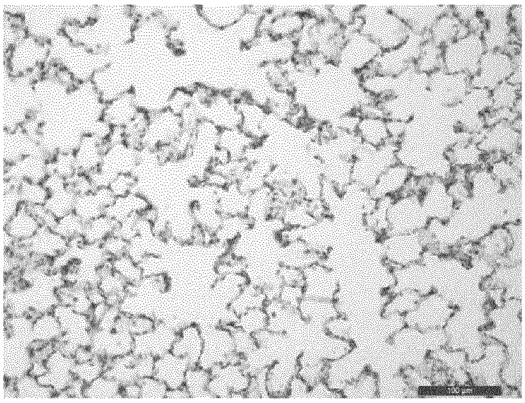
Fig. 48

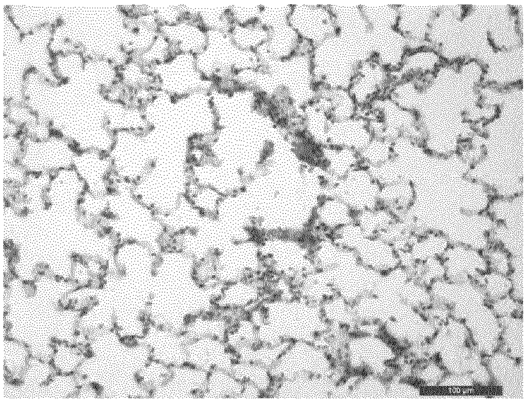

Fig. 49

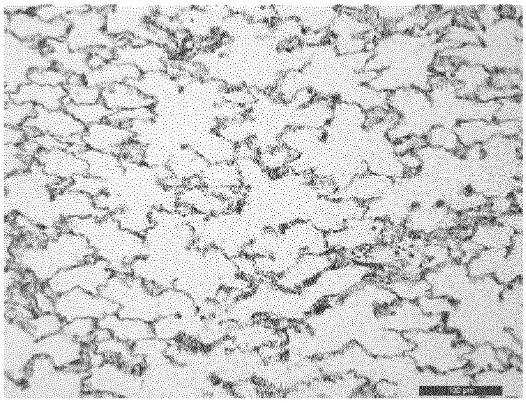

Fig. 50

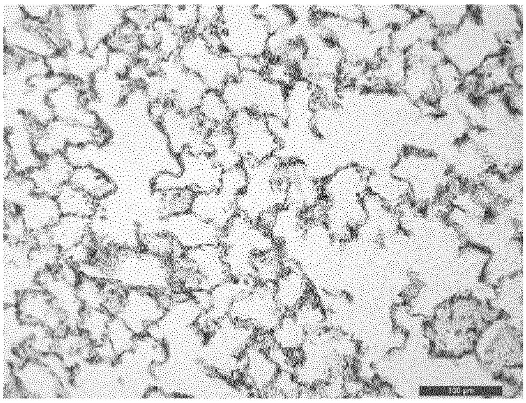

Fig. 51

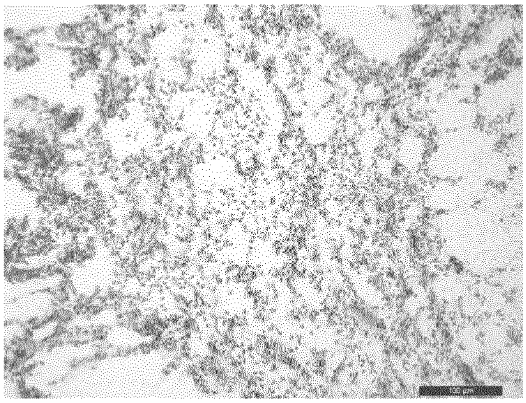

Fig. 52

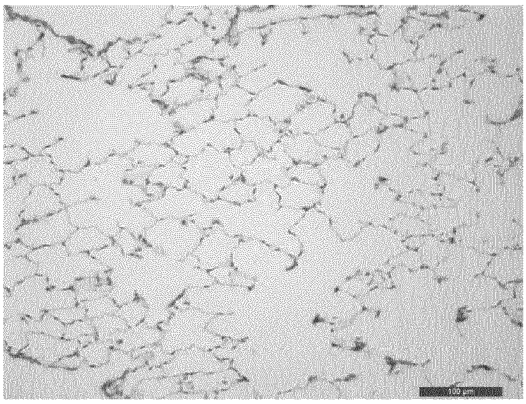

Fig. 53

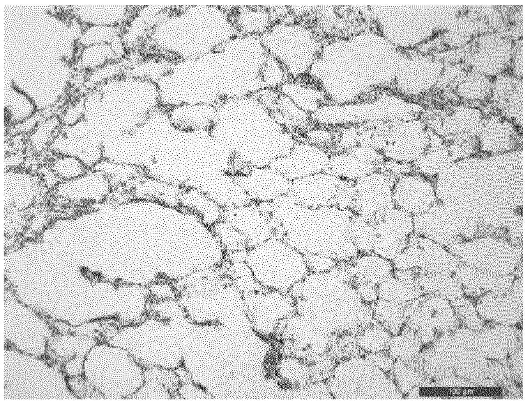

Fig. 54

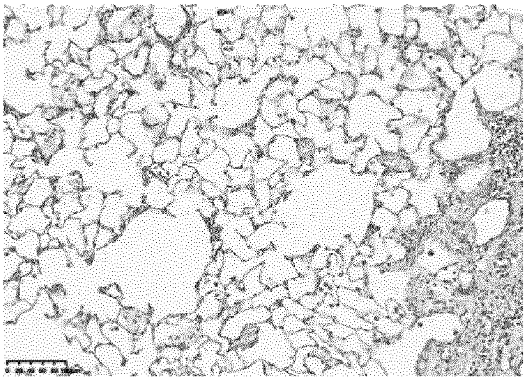
Fig. 55

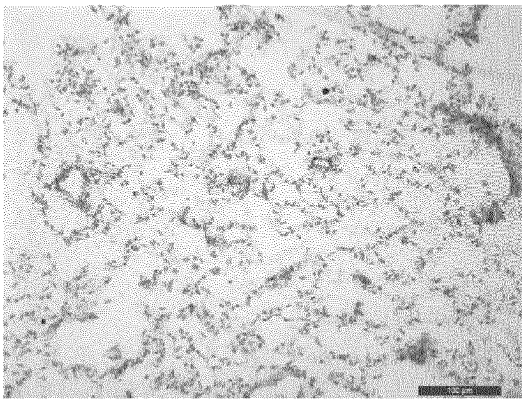
Fig. 56

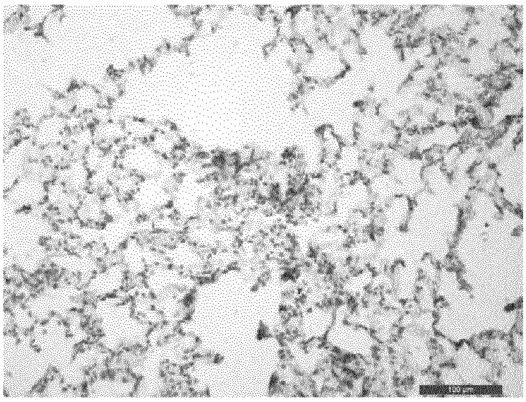
Fig. 57

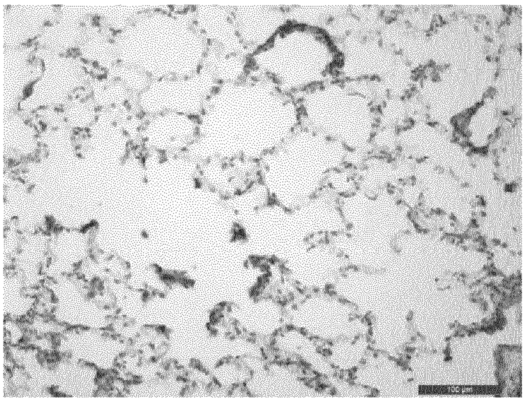
Fig. 58

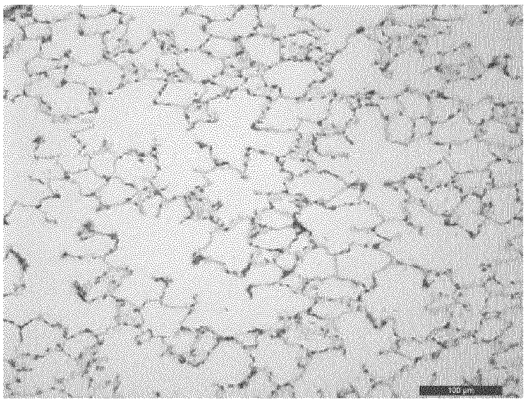
Fig. 59

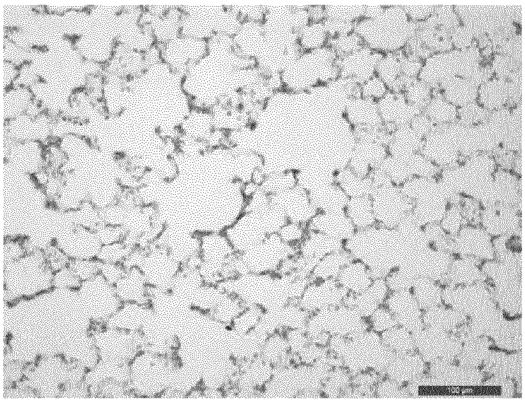
Fig. 60

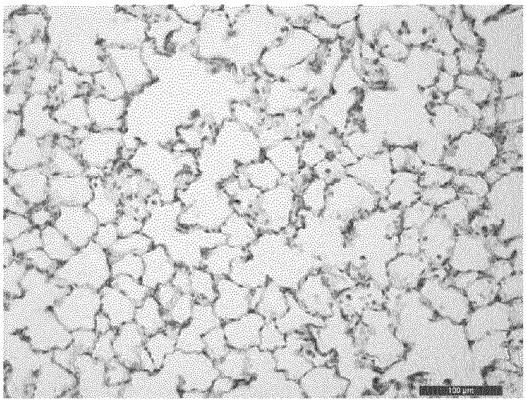

Fig. 61

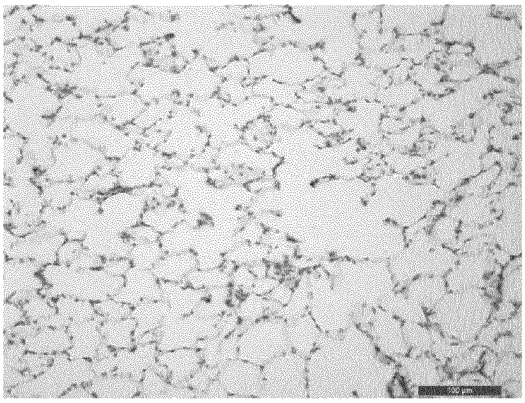

Fig. 62

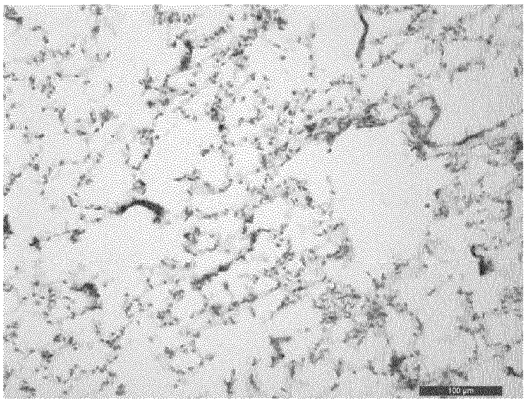

Fig. 63

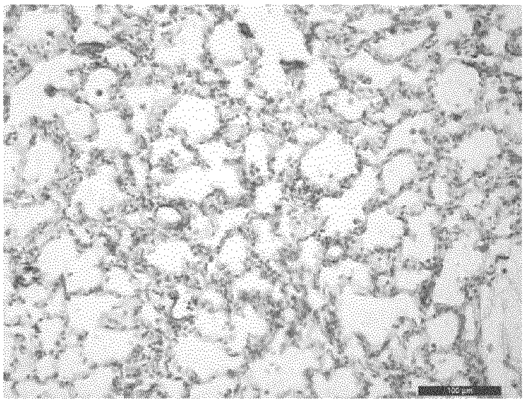

Fig. 64

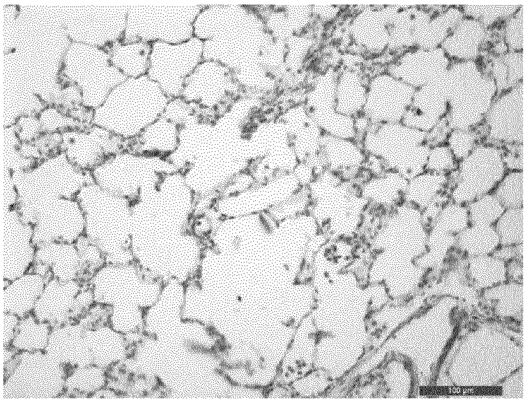

Fig. 65

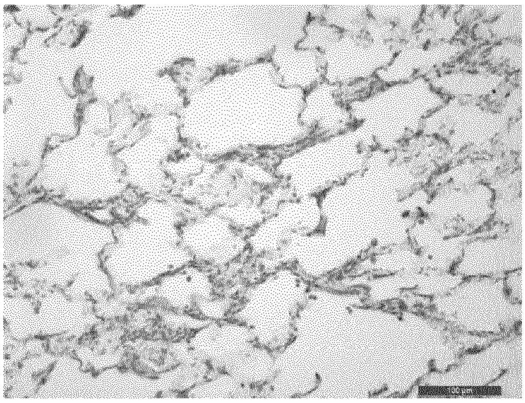

Fig. 66

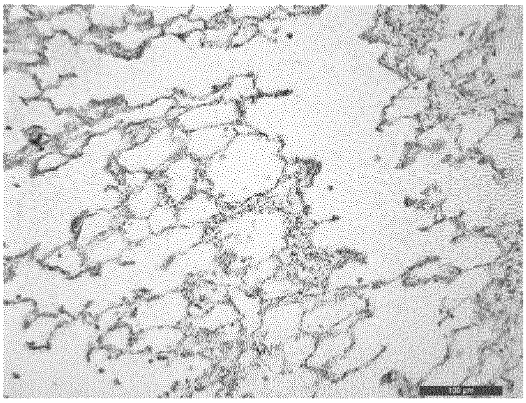

Fig. 67

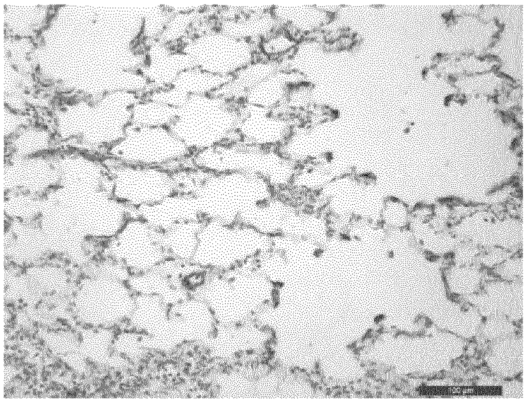

Fig. 68

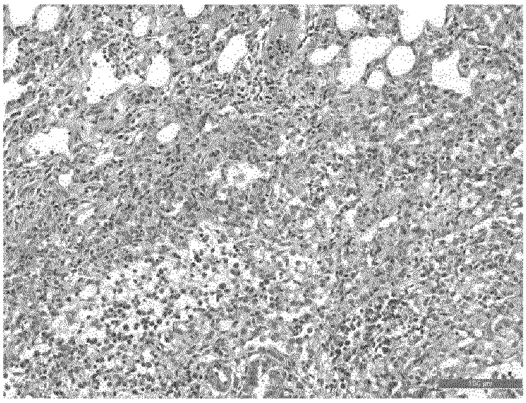

Fig. 69

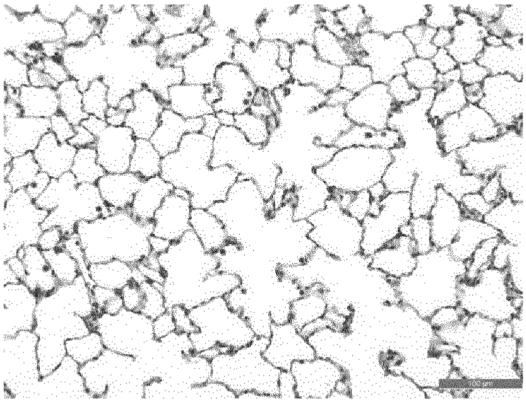

Fig. 70

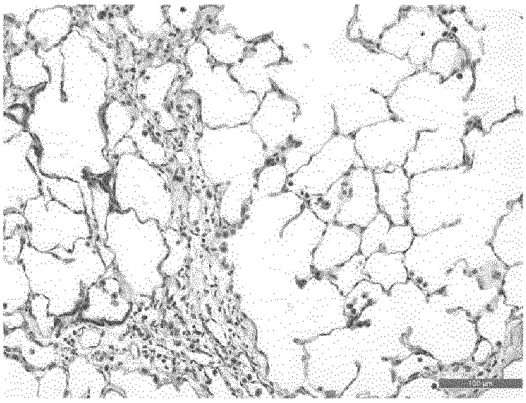

Fig. 71

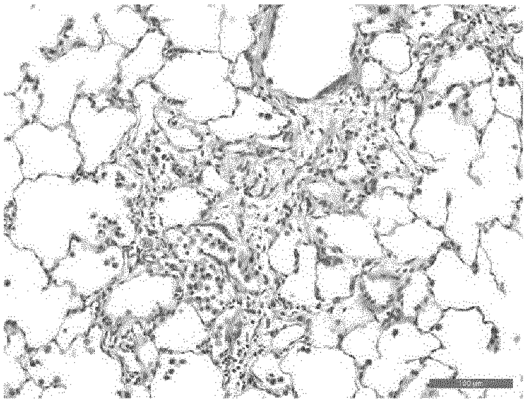

Fig. 72

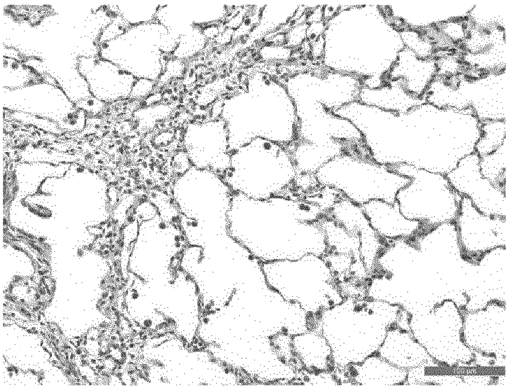

Fig. 73

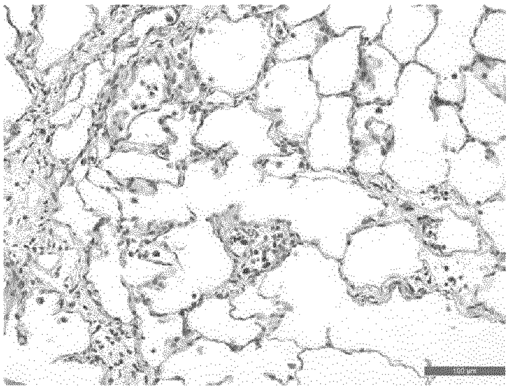

Fig. 74

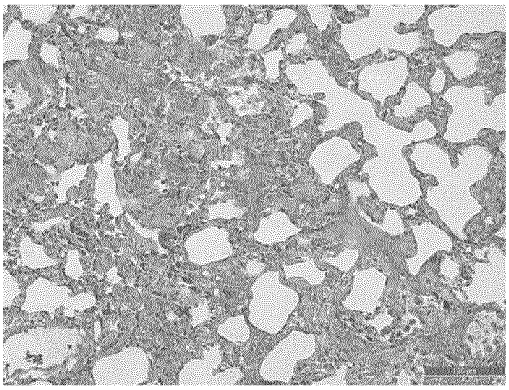

Fig. 75

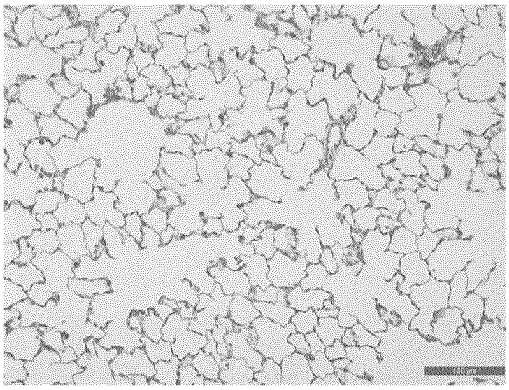

Fig. 76

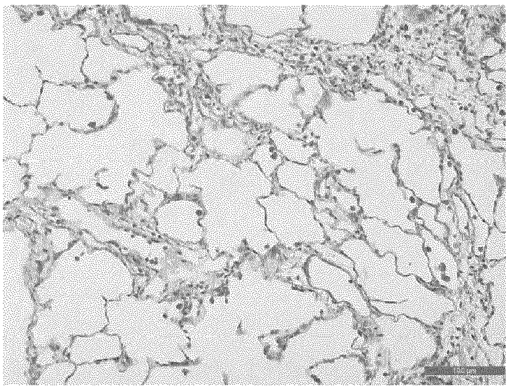

Fig. 77

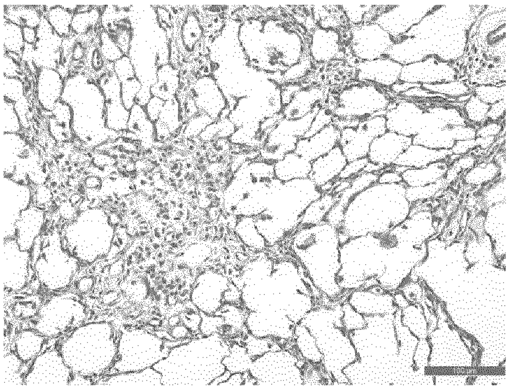

Fig. 78

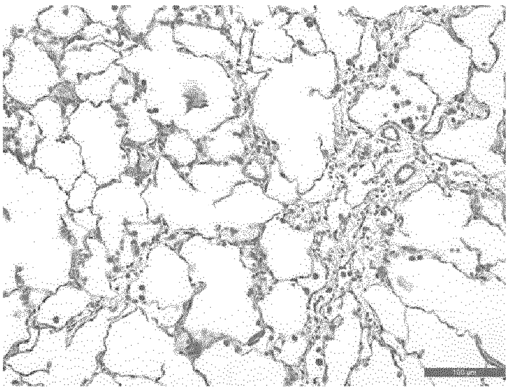

Fig. 79

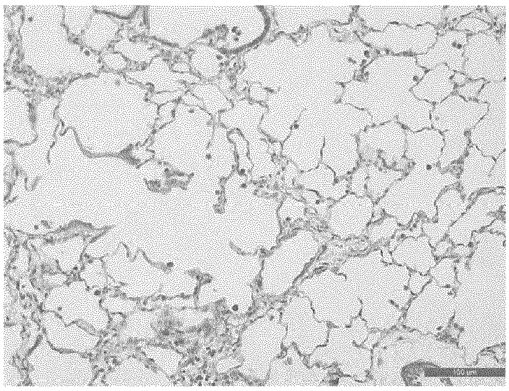

Fig. 80

## INTERNATIONAL SEARCH REPORT

International application No.
**PCT/CN2023/123369**

**A. CLASSIFICATION OF SUBJECT MATTER**

A61K 31/7048(2006.01)i; A61K 31/122(2006.01)i; A61P 11/00(2006.01)i

According to International Patent Classification (IPC) or to both national classification and IPC

**B. FIELDS SEARCHED**

Minimum documentation searched (classification system followed by classification symbols)

IPC:A61K 31/-; A61P 11/-

Documentation searched other than minimum documentation to the extent that such documents are included in the fields searched

Electronic data base consulted during the international search (name of data base and, where practicable, search terms used)

CNABS; CNTXT; VEN; WOTXT; USTXT; EPTXT; GBTXT; CATXT; CNKI; 万方, WANFANG; STN; WEB OF SCIENCE: 弥漫性肺间质纤维化; 特发性肺纤维化; 芍药苷; 异甘草素; 河南中医药大学; 李建生; 田燕歌; 冯素香; 赵鹏; 任周新; 余学庆; 刘学芳; 董浩然; 杨曙光; diffuse interstitial fibrosis; pulmonary interstitial fibrosis; interstitial pulmonary fibrosis; idiopathic pulmonary fibrosis; IPF; paeoniflorin; isoliquiritigenin; cas: 23180-57-6; cas: 961-29-5

**C. DOCUMENTS CONSIDERED TO BE RELEVANT**

| Category* | Citation of document, with indication, where appropriate, of the relevant passages | Relevant to claim No. |
|---|---|---|
| X | CN 104189006 A (INSTITUTE OF BASIC THEORY FOR CHINESE MEDICINE, CHINA ACADEMY OF CHINESE MEDICAL SCIENCES) 10 December 2014 (2014-12-10)<br>claim 10 | 6 |
| A | CN 109394776 A (HENAN UNIVERSITY OF CHINESE MEDICINE) 01 March 2019 (2019-03-01)<br>claim 1 | 1-6 |
| A | CN 102488699 A (CHINA PHARMACEUTICAL UNIVERSITY) 13 June 2012 (2012-06-13)<br>entire document | 1-6 |
| A | CN 114272254 A (SHANGHAI INSTITUTE OF MATERIA MEDICA, CHINESE ACADEMY OF SCIENCES) 05 April 2022 (2022-04-05)<br>entire document | 1-6 |
| A | US 2009074708 A1 (OLIVER YOA-PU HU et al.) 19 March 2009 (2009-03-19)<br>entire document | 1-6 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **07.04月2024（07.04.2024）** | **16 April 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2023/123369**

**C. DOCUMENTS CONSIDERED TO BE RELEVANT**

| Category* | Citation of document, with indication, where appropriate, of the relevant passages | Relevant to claim No. |
|---|---|---|
| A | 李建生等 (LI, Jiansheng et al.). "金水缓纤方治疗肺纤维化的组分配伍研究 (Effective Component Compatibility-Based Analysis of Jinshui Huanxian Formula for Treatment of Pulmonary Fibrosis)" *中草药 (Chinese Traditional and Herbal Drugs),* Vol. 52, No. 13, 31 July 2021 (2021-07-31), pages 3966-3979 ISSN: 0253-2670, pages 3966-3979 | 1-6 |

Form PCT/ISA/210 (second sheet) (July 2022)

**INTERNATIONAL SEARCH REPORT**
**Information on patent family members**

International application No.
**PCT/CN2023/123369**

| Patent document cited in search report | | | Publication date (day/month/year) | Patent family member(s) | | | Publication date (day/month/year) |
|---|---|---|---|---|---|---|---|
| CN | 104189006 | A | 10 December 2014 | None | | | |
| CN | 109394776 | A | 01 March 2019 | None | | | |
| CN | 102488699 | A | 13 June 2012 | None | | | |
| CN | 114272254 | A | 05 April 2022 | None | | | |
| US | 2009074708 | A1 | 19 March 2009 | US | 2016184298 | A1 | 30 June 2016 |
| | | | | US | 9415034 | B2 | 16 August 2016 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 109394776 B


### Non-patent literature cited in the description


- *CHEMICAL ABSTRACTS*, 961-29-5
- *CHEMICAL ABSTRACTS*, 489-32-7
- *CHEMICAL ABSTRACTS*, 478-01-3
- *CHEMICAL ABSTRACTS*, 23496-41-5